(19) Europäisches Patentamt
European Patent Office
Office européen des brevets

(11) **EP 1 270 725 B1**

(12) **EUROPÄISCHE PATENTSCHRIFT**

(45) Veröffentlichungstag und Bekanntmachung des Hinweises auf die Patenterteilung:
**19.01.2011 Patentblatt 2011/03**

(51) Int Cl.:
*C12N 15/12* *(2006.01)*    *C12N 15/62* *(2006.01)*
*C12N 15/86* *(2006.01)*    *G01N 33/68* *(2006.01)*

(21) Anmeldenummer: **02020399.8**

(22) Anmeldetag: **25.09.1998**

(54) **Anticaline**

Anticalines

Anticalines

(84) Benannte Vertragsstaaten:
**AT BE CH DE DK FI FR GB IT LI NL SE**

(30) Priorität: **26.09.1997 DE 19742706**

(43) Veröffentlichungstag der Anmeldung:
**02.01.2003 Patentblatt 2003/01**

(62) Dokumentnummer(n) der früheren Anmeldung(en) nach Art. 76 EPÜ:
**98954239.4 / 1 017 814**

(73) Patentinhaber: **Pieris AG**
**85354 Freising (DE)**

(72) Erfinder:
• **Skerra, Arne**
**85354 Freising (DE)**
• **Beste, Gerald**
**64283 Darmstadt (DE)**
• **Schmidt, Frank**
**60598 Frankfurt (DE)**
• **Stibora, Thomas**
**64331 Weiterstadt (DE)**

(74) Vertreter: **Viering, Jentschura & Partner**
**Postfach 22 14 43**
**80504 München (DE)**

(56) Entgegenhaltungen:
**WO-A-96/23879**

• **MULLER H N ET AL: "Functional expression of the uncomplexed serum retinol-binding protein in Escherichia coli. Ligand binding and reversible unfolding characteristics" JOURNAL OF MOLECULAR BIOLOGY, (1993 APR 5) 230 (3) 725-32. JOURNAL CODE: J6V. ISSN: 0022-2836., XP002095121 ENGLAND: United Kingdom**
• **MUELLER, HOLGER N. ET AL: "Grafting of a High-Affinity Zn(II)- Binding Site on th.beta.-Barrel of Retinol - Binding Protein Results in Enhanced Folding Stability and Enables Simplified Purification" BIOCHEMISTRY (1994), 33(47), 14126-35 CODEN: BICHAW;ISSN: 0006-2960, XP002095122**
• **SCHMIDT F S ET AL: "The bilin-binding protein of Pieris brassicae. cDNA sequence and regulation of expression reveal distinct features of this insect pigment protein." EUROPEAN JOURNAL OF BIOCHEMISTRY, (1994 FEB 1) 219 (3) 855-63. JOURNAL CODE: EMZ. ISSN: 0014-2956., XP002095123 GERMANY: Germany, Federal Republic of**
• **SIVAPRASADARAO A. ET AL: "Lipocalin structure and function." BIOCHEM. SOC. TRANS., (1993) 21/3 (619-622). ISSN: 0300-5127 CODEN: BCSTB5, XP002095124 United Kingdom**
• **FLOWER D.: "Multiple molecular recognition properties of the lipocalin proteinfamily" JOURNAL OF MOLECULAR RECOGNITION, Bd. 8, 1995, Seiten 185-195, XP002095125**
• **FLOWER D.: "The lipocalin protein family: structure and function" BIOCHEMICAL JOURNAL, Bd. 318, 1996, Seiten 1-14, XP002095126**

- **SUNDARAM M. ET AL.: "Expression, characterization and engineered specificity of rat epididymal retinoic-acid binding protein" BIOCHEMICAL JOURNAL, Bd. 334, Nr. 1, 15. August 1998 (1998-08-15), Seiten 155-160, XP002095127**

**Beschreibung**

Die vorliegende Erfindung betrifft neue Polypeptide, Verfahren zu ihrer Herstellung sowie ihre Verwendung zur Bindung oder Erkennung vorgegebener Liganden.

Die Lipocaline (Pervaiz and Brew, FASEB J. 1 (1987), 209-214) sind eine Familie kleiner, oft monomerer sekretorischer Proteine, die aus unterschiedlichen Organismen isoliert wurden, und deren physiologische Rolle in der Speicherung oder dem Transport verschiedener Liganden, wie auch in komplexeren biologischen Funktionen besteht (Flower, Biochem. J. 318 (1996), 1-14). Die Lipocaline weisen untereinander relativ geringe Sequenzähnlichkeit auf, und ihre Zugehörigkeit zu derselben Proteinstrukturfamilie wurde erst durch die Röntgenstrukturanalyse aufgedeckt (Sawyer et al., Nature 327 (1987), 659).

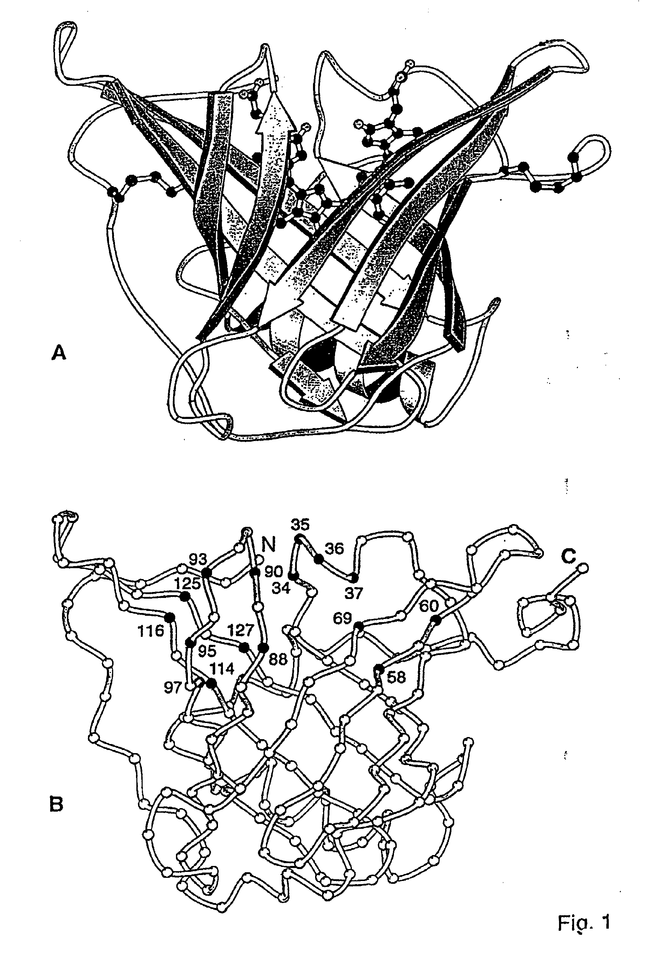

Das erste Lipocalin mit bekannter Raumstruktur war das menschliche Retinol-Bindungsprotein, Rbp, das den Transport des wasserunlöslichen Vitamin A im Blutserum bewirkt (Newcomer et al., EMBO J. 3 (1984), 1451-1454). Kurze Zeit später wurde die Tertiärstruktur des Bilin-Bindungsproteins, Bbp, aus dem Schmetterling *Pieris brassicae* aufgeklärt (Huber et al., J. Mol. Biol. 195 (1987), 423-434). Anhand der Raumstruktur dieses Lipocalins, die in Figur 1A schematisch wiedergegeben ist, lassen sich die wesentlichen Strukturmerkmale dieser Proteinklasse erläutern. Zentrales Element in der Faltungsarchitektur der Lipocaline ist die zylindrische Faltblattstruktur, das sogenannte β-Barrel, die sich aus acht nahezu kreisförmig angeordneten antiparallelen β-Faltblattsträngen zusammensetzt.

Dieses Supersekundärstrukturelement läßt sich auch als "Sandwich"-Anordnung zweier viersträngiger β-Faltblattstrukturen auffassen. Zusätzliche Strukturelemente sind ein gestrecktes Segment am Aminoterminus der Polypeptidkette und eine α-Helix in der Nähe des Carboxyterminus, die wiederum von einem gestreckten Segment gefolgt ist. Diese zusätzlichen Merkmale sind jedoch nicht notwendigerweise in allen Lipocalinen ausgeprägt. So fehlt z. B. ein erheblicher Teil des N-terminalen Segments in dem epididymalen Retinsäure-Bindungsprotein (Newcomer, Structure 1 (1993), 7-18). Ferner sind auch zusätzliche, spezielle Strukturelemente bekannt, wie beispielsweise Membrananker (Bishop und Weiner, Trends Biochem. Sci. 21 (1996), 127), die nur in bestimmten Lipocalinen vorkommen.

An einem Ende ist das β-Barrel durch dichte Aminosäurepackung sowie durch Schleifensegmente verschlossen. Am anderen Ende bildet das β-Barrel eine Bindungstasche, in der der jeweilige Ligand des Lipocalins komplexiert wird. Dort sind die acht benachbarten antiparallelen β-Faltblattstränge jeweils paarweise durch Kehren in der Polypeptidkette verbunden, die zusammen mit den angrenzenden Aminosäuren, die sich teilweise noch im Bereich der zylindrischen Faltblattstruktur befinden, jeweils ein Schleifensegment bilden. Die Bindungstasche für den Liganden wird von diesen insgesamt vier Peptidschleifen gebildet. Im Fall des Bbp wird das Biliverdin IXγ in dieser Bindungstasche komplexiert. Ein anderer typischer Ligand für Lipocaline ist das Vitamin A im Fall des Rbp wie auch des β-Lactoglobulins (Papiz et al., Nature 324 (1986), 383-385).

Gegenüberstellungen der Sequenzen von verschiedenen Vertretern der Lipocalinfamilie sind unter anderem in der Veröffentlichung von Cowan et al. (Proteins: Struct., Funct., Genet. 8 (1990), 44-61) und in dem Übersichtsartikel von Flower (FEBS Lett. 354 (1994), 7-11) zu finden. Unter den derzeit weit mehr als 20 unterschiedlichen bekannten Lipocalinen befinden sich vor allem zwei menschliche Proteine, die bereits näher biochemisch charakterisiert wurden: das Retinol-Bindungsprotein und das Apolipoprotein D, ApoD (Yang et al., Biochemistry 33 (1994), 12451-12455). Das ApoD ist besonders interessant, da es enge strukturelle Verwandtschaft mit dem oben erwähnten Bbp aufweist (Peitsch und Boguski, New Biologist 2 (1990), 197-206).

Ein klassisches Beispiel für Proteine, die mittels nicht kovalenter Wechselwirkungen Liganden selektiv binden, stellen die Antikörper, d. h. Immunglobuline, dar. Diese Proteine spielen als Reagenzien auf den Gebieten der Biotechnologie, der Medizin, der Bioanalytik sowie ganz allgemein in den Biowissenschaften eine herausragende Rolle. Trotz der Vielfalt der gegebenen Einsatzmöglichkeiten im Zusammenhang mit der Erkennung, Bindung oder Abtrennung von Liganden werden heute beinahe ausschließlich Immunglobuline für entsprechende Zwecke eingesetzt. Die Anwendung anderer Proteine mit definierten Liganden-Bindungseigenschaften, wie z. B. der Lektine, ist dagegen auf Spezialfälle beschränkt geblieben.

Spezifische Antikörper lassen sich gegen verschiedenartigste Zielstrukturen, sogenannte Haptene bzw. Antigene, gezielt herstellen. Neben dem inzwischen allgemein etablierten Verfahren zur Produktion monoklonaler Antikörper werden dazu neuerdings auch biosynthetische Methoden eingesetzt, beispielsweise unter Verwendung der "Phage Display"-Technik (Hoess, Curr. Opin. Struct. Biol. 3 (1993), 572-579; Wells and Lowman, Curr. Opin. Struct. Biol. 2 (1992), 597-604). Ist erst einmal die genetische Information für die Bindungsregion (variable Domänen VH und VL) eines Immunglobulins mit der gewünschten Hapten- oder Antigenspezifität bekannt, so stehen dem Fachmann für die Produktion dieses Antikörpers, seiner Fragmente oder davon abgeleiteter Hybridproteine zahlreiche gentechnische Verfahren unter Verwendung von eukaryontischen oder bakteriellen Expressionssystemen zur Verfügung. Dennoch zeichnen sich mitunter Nachteile beim praktischen Einsatz dieser Proteinklasse ab.

Beispielsweise ist es bei medizinischen Anwendungen wie z. B. dem "Tumor Imaging" oder dem "Drug Targeting" (Chester und Hawkins, Trends Biotechnol. 13 (1995) 294-300) wünschenswert, möglichst kleine Bindungsdomänen einzusetzen, da man sich davon eine verbesserte Gewebepenetration verspricht. Nach allgemeiner Ansicht ist das Fv-Fragment, welches sich aus der variablen Domäne der leichten Polypeptidkette (VL) und der variablen Domäne der schweren Polypeptidkette (VH) eines Antikörpers zusammensetzt, in der Regel das kleinste Immunglobulinfragment, welches eine strukturell intakte Antigen-Bindungsstelle ausbildet. Typischerweise besteht ein Fv-Fragment allerdings aus ungefähr 240 Aminosäuren, so daß ein solches Protein immer noch verhältnismäßig große Moleküldimensionen aufweist.

Des weiteren kann der Aufbau der Antikörper aus zwei verschiedenen Polypeptidketten (leichte und schwere Kette) zu unerwünschten Effekten führen. Da jeweils ein Paar kodierender Regionen kloniert und ggf. exprimiert werden muß, ist die gentechnische Produktion und Handhabung im Vergleich zu Proteinen aus einer einzelnen Polypeptidkette erschwert. Zudem hat sich gezeigt, daß Fv-Fragmente nicht selten über geringe proteinchemische Stabilität verfügen, da ihre VL- und VH-Domänen bloß über nicht kovalente Wechselwirkungen aneinander gebunden sind. Mit verschiedenen Strategien wurde daher schon versucht, die Assoziation der beiden variablen Domänen in dem heterodimeren Fv-Fragment zu stabilisieren. Eine dieser Methoden bedient sich der Verknüpfung der beiden Polypeptidketten auf der Ebene der Translation, wobei sogenannte scFv-Fragmente (Bird und Walker, Trends Biotechnol. 9 (1991), 132-137) erhalten werden. Allerdings hat sich gezeigt, daß diese Vorgehensweise mitunter andere Nachteile mit sich bringt, wie zum Beispiel Einbußen in der Affinität für den Liganden oder ein unerwünschtes Oligomerisierungsverhalten (Desplancq et al., Protein Eng. 7 (1994), 1027-1033).

Der Erfindung liegt deshalb die Aufgabe zugrunde, andere Polypeptidreagenzien, die wie die Antikörper spezifische Bindungseigenschaften für vorgegebene Liganden aufweisen, zu entwickeln. Erfindungsgemäß wird diese Aufgabe gelöst mit einem Lipocalinmutein, basierend auf einem Polypeptid der Lipocalinfamilie. Bei diesem Lipocalinmutein sind Aminosäurepositionen im Bereich von drei der vier Peptidschleifen, die an einem Ende der zylindrischen Faltblattstruktur angeordnet sind, und die denjenigen Abschnitten in der linearen Polypeptidsequenz entsprechen, die die Aminosäurepositionen 28 bis 45, 58 bis 69, 86 bis 99 und 114 bis 129 des Bilin-Bindungsproteins (Bbp) aus *Pieris brassicae* umfassen, mutiert. Das Lipocalinmutein bindet einen vorgegebenen neuen Liganden mit bestimmbarer Affinität. Lipocalinmuteine sind herstellbar ausgehend von Polypeptiden der Lipocalinfamilie, indem Aminosäuren im Bereich der vier Peptidschleifen, die an einem Ende der zylindrischen Faltblattstruktur angeordnet sind, mutiert werden, und die dadurch charakterisiert sind, daß sie einen vorgegebenen Liganden mit bestimmbarer Affinität binden.

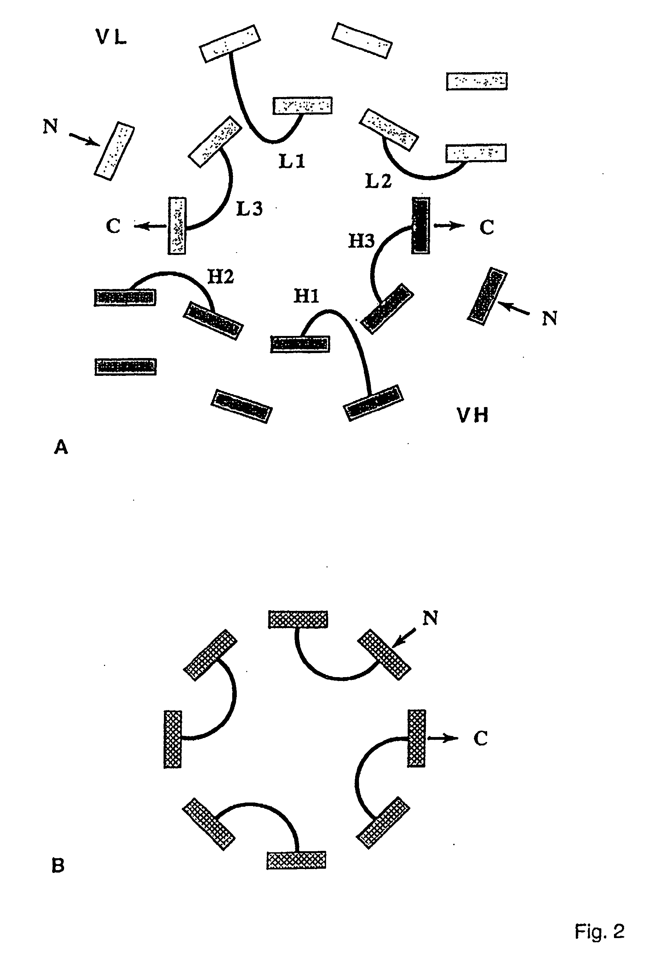

Ein topographischer Vergleich des Verlaufs der Polypeptidkette in der Proteinfaltung der Lipocaline mit den Fv-Fragmenten der Immunglobuline wird aus Figur 2 ersichtlich. In den Immunglobulinen wird die Bindungsstelle für das Antigen von sechs strukturell hypervariablen Peptidschleifen, auch CDRs (engl.: Complementarity Determining Regions) genannt, gebildet. Beide variable Domänen, VH und VL, tragen drei CDRs zur Antigen-Bindungsstelle bei. Die beiden variablen Domänen bestehen jeweils aus zwei schichtartig angeordneten β-Faltblattstrukturen, die das strukturell konservierte Gerüst bilden, welches die hypervariablen Peptidschleifen trägt. In dem Fv-Fragment entsteht so ein innerer und ein äußerer Ring von β-Faltblattsträngen, wobei zwei CDRs zwischen benachbarten Strängen des inneren Rings und vier CDRs zwischen Strängen des inneren und des äußeren Rings aufgespannt sind.

Im Vergleich dazu sind die Liganden-Bindungsstellen der Lipocaline einfacher aufgebaut. In diesem Fall liegt nur ein Ring von 8 antiparallelen β-Faltblattsträngen vor: das β-Barrel. Diese zyklische Faltblattstruktur ist in der Proteinfaltung der Lipocaline konserviert. Die Bindungsstelle wird im Eingangsbereich des β-Barrels von den vier Peptidschleifen gebildet, die jeweils zwei benachbarte β-Faltblattstränge miteinander verbinden. Diese Peptidschleifen können sich in ihrer Struktur erheblich zwischen den einzelnen Mitgliedern der Lipocalinfamilie unterscheiden.

Trotz der scheinbaren Analogie im strukturellen Aufbau der Immunglobuline und der Lipocaline, d. h. konservierte Gerüstbereiche einerseits und hypervariable, spezifitätsbestimmende Abschnitte andererseits, gibt es einen wesentlichen Unterschied zwischen diesen beiden Proteinklassen. Während nämlich im menschlichen Körper ca. 100 Millionen verschiedene Antikörper zirkulieren und ständig neu gebildet werden, bringt derselbe Organismus nur wenige verschiedene Lipocaline - wie z. B. das oben erwähnte Rbp oder das ApoD - hervor. Entstehen im Immunsystem eines Säugetiers durch somatische Genrekombination und Mutation fortwährend Antikörper mit neuen Antigenspezifitäten, so sind die Lipocaline im Gegensatz dazu im Verlauf der Evolution in der Struktur und Funktion ihrer jeweiligen Liganden-Bindungsstellen weitestgehend konserviert geblieben. Als Beispiel dafür kann das Rbp dienen, dessen Aminosäuresequenz aus verschiedenen Organismen bekannt ist. Der Sequenzvergleich mit dem menschlichen Rbp (SWISS-PROT Datenbank-Zugriffscode P02753) zeigt, daß beispielsweise zu dem Rbp des Schweins (SWISS-PROT Datenbank-Zugriffscode P27485) bloß 13 und zu dem des Rinds (SWISS-PROT Datenbank-Zugriffscode P18902) bloß 14 Unterschiede bestehen. Alle diese Aminosäuresubstitutionen befinden sich zudem in der Raumstruktur fernab der Bindungsstelle für das Retinol (s. die Figur 13 in der Veröffentlichung von Cowan et al., supra).

In dem erfindungsgemäßen Verfahren wird diese Lücke zwischen den funktionellen Eigenschaften der Antikörper und der Lipocaline geschlossen, indem drei der vier Peptidschleifen, die die Liganden-Bindungsstelle eines Lipocalins bilden, einer Mutagenese unterzogen werden und im Anschluß daran solche Proteinvarianten (Muteine) ausgewählt, d. h. selektiert werden, die die gewünschte Bindungsaktivität für einen vorgegebenen Liganden aufweisen. Die dabei erhaltenen Lipocalinmuteine werden hier auch als Anticaline bezeichnet.

Im folgenden wird an einem Beispiel, nämlich dem Bbp, erläutert, was unter dem Begriff Peptidschleifen in dieser Erfindung anhand der Polypeptidsequenz verstanden werden soll. Die vier Peptidschleifen der Lipocaline, die bei der erfindungsgemäßen Herstellung der Lipocalinmuteine durch Mutagenese in ihrer Sequenz abgewandelt werden, sind durch diejenigen Abschnitte in der linearen Polypeptidsequenz gekennzeichnet, die die Aminosäurepositionen 28 bis 45, 58 bis 69, 86 bis 99 und 114 bis 129 des Bbp umfassen. Diese Sequenzabschnitte beginnen jeweils vor dem C-Terminus eines der konservierten β-Faltblattstränge an der offenen Seite des β-Barrels, schließen die eigentliche Peptidkehre ein, und enden nach dem N-Terminus des in der Sequenz folgenden, ebenfalls konservierten β-Faltblattstrangs.

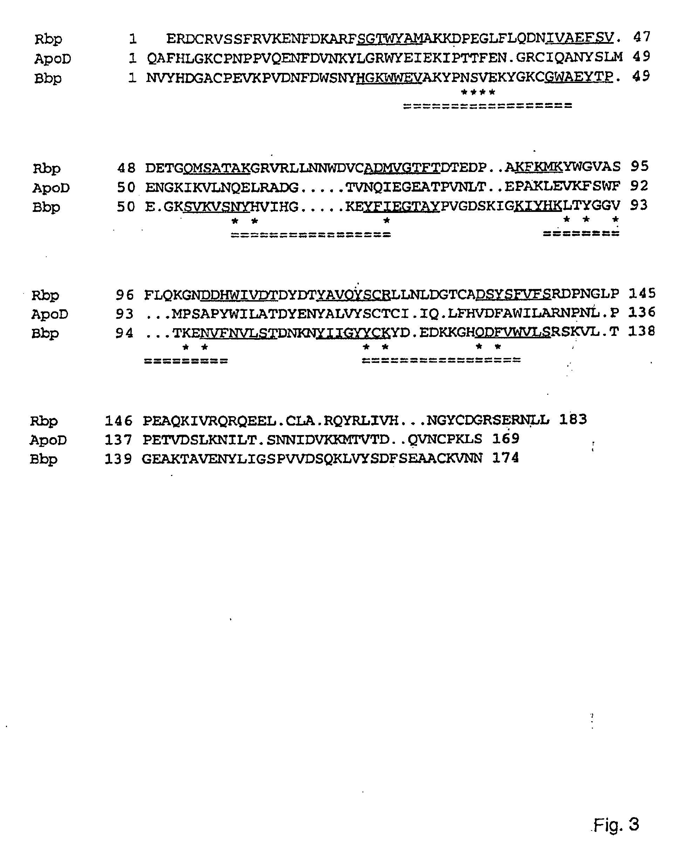

Anhand veröffentlichter oder vom Fachmann selbst durchführbarer Sequenz-Gegenüberstellungen (Alignments) oder Strukturüberlagerungen läßt sich die Definition der für das Bbp angegebenen Sequenzpositionen auf andere Lipocaline übertragen. Beispielsweise kann man aus der in Figur 3 wiedergegebenen Sequenz-Gegenüberstellung, die dem von Peitsch und Boguski (New Biologist 2 (1990), 197-206) veröffentlichten Alignment entspricht, ablesen, daß die vier Peptidschleifen im Fall des ApoD die Aminosäurepositionen 28 bis 44, 59 bis 70, 85 bis 98 und 113 bis 127 umfassen. Mit der beschriebenen Vorgehensweise ist es möglich, auch in neuen Lipocalinen die entsprechenden Peptidschleifen zu identifizieren, die sich für eine erfindungsgemäße Mutagenese eignen.

Als problematisch bei der Ermittlung der konservierten β-Faltblattstränge kann sich in manchen Fällen die relativ schwach ausgeprägte Sequenzhomologie der Lipocaline erweisen. Entscheidend ist daher die Fähigkeit der Polypeptidsequenz, die zyklische Faltblattstruktur aus 8 antiparallelen β-Faltblattsträngen auszubilden. Diese läßt sich ggf. unter Einsatz strukturanalytischer Methoden wie der Proteinkristallographie oder der multidimensionalen Kernresonanz-Spektroskopie nachweisen.

Die zur Mutagenese geeigneten Sequenzabschnitte können bei anderen Lipocalinen, wie z. B. dem ApoD oder dem Rbp, aufgrund der jeweils variierenden Struktur der Peptidschleifen durchaus länger oder kürzer sein als beim Bbp (vgl. Figur 3). Es kann sogar von Vorteil sein, einen oder mehrere der Sequenzabschnitte durch Deletion oder Insertion von einer oder mehreren Aminosäuren zusätzlich in seiner Länge zu verändern. In einer bevorzugten Ausführung der Erfindung werden diejenigen Aminosäurepositionen in diesen Sequenzabschnitten, die den Sequenzpositionen 34 bis 37, 58, 60, 69, 88, 90, 93, 95, 97, 114, 116, 125 und 127 des Bbp entsprechen, und die in den Figuren 1B und 3 hervorgehoben sind, mutiert. Im Fall des ApoD sind demgemäß die Sequenzpositionen 34 bis 37, 59, 61, 70, 87, 89, 92, 94, 96, 113, 115, 123 und 125 für die Mutagenese bevorzugt. Für die Herstellung von Lipocalinmuteinen müssen jedoch nicht alle hier angegebenen Sequenzpositionen einer Mutagenese unterzogen werden.

Als Grundstruktur zur Herstellung von Lipocalinmuteinen sind selbstverständlich neben den hier genannten Beispielen auch andere Lipocaline geeignet. Bevorzugt sind die zur Zeit bereits sehr gründlich biochemisch untersuchten Lipocaline Rbp, Bbp oder ApoD zu verwenden. Besonders bevorzugt sind Lipocaline humanen Ursprungs zur Herstellung von Anticalinen zu verwenden. Dies gilt, vor allem, wenn eine Anwendung des oder der resultierenden Lipocalinmuteine am Menschen beabsichtigt ist, da beispielsweise bei diagnostischen oder therapeutischen Anwendungen in vivo im Vergleich zu den Lipocalinen aus anderen Organismen minimale immunogene Wirkung zu erwarten ist. Jedoch können sich auch andere und ggf. künftig erst neu zu entdeckende Lipocaline als besonders vorteilhaft zur Herstellung von Lipocalinmuteinen erweisen. Ebenso können künstliche Proteine mit einem dem β-Barrel der Lipocaline strukturell äquivalenten Faltungselement dazu verwendet werden. Vorzugsweise sollen die erfindungsgemäßen Lipocalinmuteine den gewünschten Liganden mit bestimmbarer Affinität, d. h. mit einer Affinitätskonstante von mindestens $10^5$ M$^{-1}$ binden. Niedrigere Affinitäten lassen sich mit den üblichen Meßmethoden in der Regel nicht mehr exakt erfassen und sind daher für praktische Anwendungen von untergeordneter Bedeutung. Besonders bevorzugt sollen die Lipocalinmuteine den gewünschten Liganden mit einer Affinität von mindestens $10^6$ M$^{-1}$, entsprechend einer Komplex-Dissoziationskonstante von 1 μM, binden. Die Bindungsaffinität eines Lipocalinmuteins zu dem gewünschten Liganden kann vom Fachmann mit einer Vielzahl von Methoden ermittelt werden, beispielsweise mit dem Verfahren der Fluoreszenztitration, durch Kompetitions-ELISA oder mittels der Oberflächen-Plasmonresonanztechnik.

Als Ausgangspunkt zur Mutagenese der Peptidschleifen kann die cDNA eines Lipocalins dienen, die mit dem Fachmann bekannten Methoden hergestellt und kloniert werden kann, wie es beispielsweise für das Bbp beschrieben wurde (Schmidt und Skerra, Eur. J. Biochem. 219 (1994), 855-863). Alternativ kann auch genomische DNA eingesetzt oder eine Gensynthese oder eine Kombination dieser Verfahren durchgeführt werden. Zur Mutagenese der Aminosäuren in den vier Peptidschleifen stehen dem Fachmann die verschiedenen bekannten Verfahren zur ortsspezifischen Mutagenese oder zur Mutagenese mittels der Polymerase-Kettenreaktion zur Verfügung. Die Mutageneseverfahren können beispielsweise dadurch gekennzeichnet sein, daß Mischungen synthetischer Oligodesoxynukleotide, die an den gewünschten Positionen degenerierte Basenzusammensetzung aufweisen, zur Einführung der Mutationen verwendet werden. Auch der Einsatz von Nukleotidbausteinen mit reduzierter Basenpaarungsspezifität, wie z. B. Inosin, kommt zur Einführung von Mutationen in den ausgewählten Sequenzabschnitten oder Aminosäurepositionen in Betracht. Im Vergleich zu den Antikörpern ist die Vorgehensweise zur Mutagenese der Liganden-Bindungsstelle vereinfacht, da bei den Lipocalinen dafür nur vier anstelle von sechs Sequenzabschnitten - entsprechend den vier oben genannten Peptidschleifen - manipuliert werden müssen.

Bei den Methoden der ortsgerichteten Zufallsmutagenese unter Einsatz von synthetischen oligodesoxynukleotiden lassen sich die betreffenden Aminosäurepositionen in der Lipocalinstruktur, die mutiert werden sollen, vorherbestimmen. Die ideale Auswahl der zu mutierenden Aminosäurepositionen kann von dem verwendeten Lipocalin einerseits und dem gewünschten Liganden andererseits abhängen. Dabei kann es sinnvoll sein, die Gesamtzahl der mutierten Aminosäurepositionen innerhalb eines Experiments so gering zu halten, daß die Sammlung der bei der Mutagenese erhaltenen Varianten, d. h. die sogenannte Bibliothek, in ihrer Gesamtheit oder wenigstens in einer repräsentativen Auswahl davon sowohl auf der Ebene der kodierenden Nukleinsäure als auch auf der Ebene der Genprodukte in ihrer kombinatorischen Komplexität möglichst vollständig realisiert werden kann.

Die zu mutierenden Aminosäurepositionen sollten sich vor allem dann sinnvoll auswählen lassen, wenn Strukturinformationen über das verwendete Lipocalin selbst, wie im Fall des Rbp und des Bbp, oder zumindest über ein Lipocalin mit ähnlicher Struktur vorliegen, wie z. B. im Fall des ApoD. Der Satz der ausgewählten Aminosäurepositionen kann außerdem von den Eigenschaften des gewünschten Liganden abhängen. Z. B. kann es im Fall eines kleinen, haptenartigen Liganden sinnvoll sein, vor allem Aminosäurepositionen am Zentrum der Liganden-Bindungstasche, also noch in oder nahe dem Bereich des β-Barrels, der Mutagenese zu unterziehen. Im Fall eines größeren, antigenartigen Liganden dagegen sollte die Mutagenese auch diejenigen Aminosäurepositionen in den Peptidschleifen betreffen, die besonders exponiert an der Proteinoberfläche angeordnet sind, und die sich eher in der Mitte der entsprechenden Sequenzabschnitte befinden. Abgesehen von einer solchen funktionellen Betrachtung kann es sich zudem als vorteilhaft erweisen, einzelne Aminosäurepositionen im Bereich der Liganden-Bindungstasche von einer Mutagenese auszunehmen, wenn diese sich beispielsweise als essentiell für die Faltungseffizienz oder -stabilität des Proteins erweisen.

Eine der zahlreichen anwendbaren Methoden zur Einführung von Mutationen im Bereich der vier Peptidschleifen eines Lipocalins basiert auf der Verwendung von vier Oligodesoxynukleotiden, die jeweils von einem der vier entsprechenden zu mutierenden Sequenzabschnitte abgeleitet sind. Bei der Herstellung dieser Oligodesoxynukleotide kann der Fachmann zur Synthese derjenigen Nukleotidtripletts, die den zu mutierenden Aminosäurepositionen entsprechen, Gemische von Nukleinsäurebausteinen einsetzen, so daß zufällig Codons bzw. Anticodons für alle Aminosäuren oder, gemäß dem genetischen Code und der Zusammensetzung dieser Mischung, für eine Auswahl der an dieser Position gewünschten Aminosäuren zustandekommen.

Beispielsweise entspricht das erste Oligodesoxynukleotid in seiner Sequenz - abgesehen von den mutierten Positionen - zumindest teilweise dem kodierenden Strang für diejenige Peptidschleife, die in der Polypeptidsequenz des Lipocalins am weitesten N-terminal liegt. Das zweite Oligodesoxynukleotid entspricht demgemäß zumindest teilweise dem nichtkodierenden Strang für den in der Polypeptidsequenz folgenden zweiten Sequenzabschnitt. Das dritte Oligodesoxynukleotid entspricht wiederum zumindest teilweise dem kodierenden Strang für den entsprechenden dritten Sequenzabschnitt. Das vierte Oligodesoxynukleotid entspricht schließlich zumindest teilweise dem nichtkodierenden Strang für den vierten Sequenzabschnitt. Mit dem ersten und zweiten Oligodesoxynukleotid sowie mit dem dritten und vierten Oligodesoxynukleotid kann jeweils eine Polymerase-Kettenreaktion unter Verwendung der für das Lipocalin kodierenden Nukleinsäure und/oder ihres Gegenstrangs als Matrize durchgeführt werden.

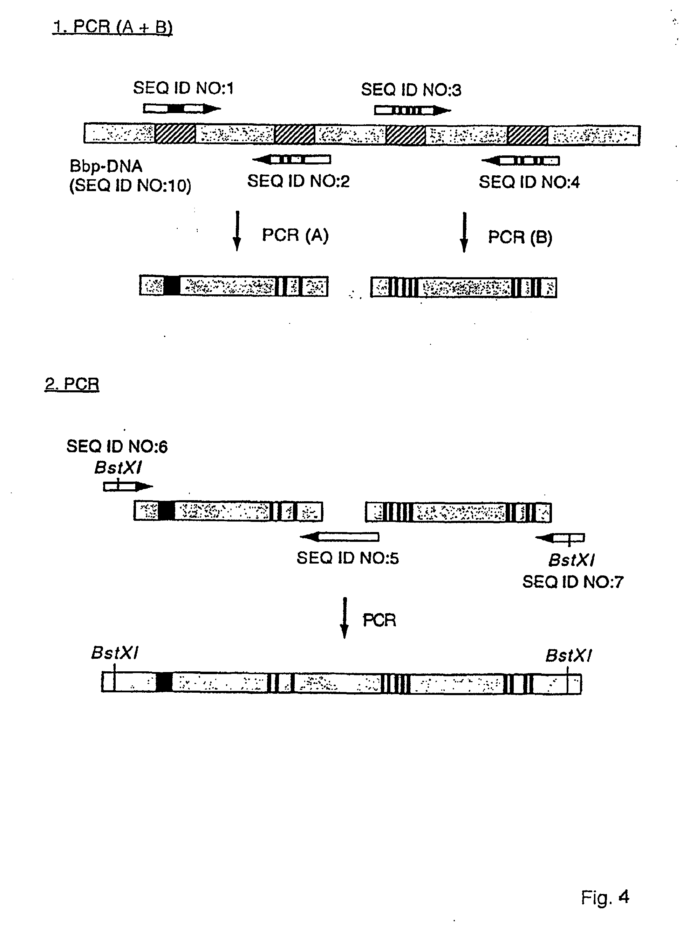

Die Amplifizierungsprodukte dieser beiden Reaktionen können durch verschiedene bekannte Methoden zu einer Nukleinsäure zusammengesetzt werden, welche die Sequenz von dem ersten bis zum vierten Sequenzabschnitt umfaßt und die Mutationen an den ausgewählten Aminosäurepositionen trägt. Beispielsweise können die beiden Produkte dazu einer erneuten Polymerase-Kettenreaktion unter Verwendung flankierender Oligodesoxynukleotide als Primer sowie eines oder mehrerer vermittelnder Nukleinsäuremoleküle, die die Sequenz zwischen dem zweiten und dem dritten Sequenzabschnitt beitragen, unterzogen werden. Diese Vorgehensweise ist in Figur 4 schematisch wiedergegeben. Bei der Wahl der Anzahl der zur Mutagenese verwendeten Oligodesoxynukleotide und deren Anordnung innerhalb der Gensequenz des Lipocalins stehen dem Fachmann darüber hinaus vielfältige Alternativen zur Verfügung.

Die Nukleinsäuremoleküle, die für den Sequenzbereich mit den vier Peptidschleifen eines Lipocalins kodieren und Mutationen an den ausgewählten Positionen enthalten, können durch Ligierung mit den fehlenden 5'- und 3'-Sequenzen einer für das Lipocalin kodierenden Nukleinsäure verbunden und in einem der bekannten Wirtsorganismen kloniert werden. Für die Ligierung und Klonierung stehen wiederum vielfältige Vorgehensweisen zur Verfügung. Beispielsweise können im Verlauf einer Amplifizierung synthetische Nukleinsäuremoleküle mit Erkennungssequenzen für Restriktionsendonukleasen, welche an den entsprechenden Positionen in der Nukleinsäuresequenz für das Lipocalin ebenfalls vorhanden sind, an den beiden Enden der zu klonierenden Nukleinsäure angefügt werden, so daß nach der Hydrolyse mit dem entsprechenden Restriktionsenzym eine Ligierung ermöglicht wird.

Die vorliegende Erfindung betrifft auch die gezielte Mutagenese einzelner Aminosäurepositionen innerhalb oder außerhalb der vier Peptidschleifen, beispielsweise um durch Einführung von Schnittstellen für bestimmte Restriktionsenzyme die Subklonierung des mutierten Lipocalingens oder seiner Teile zu vereinfachen. Beispielsweise können in das Bbp-Gen die Mutationen Asn21 zu Gln und Lys135 zu Met eingeführt werden, um die Klonierung des mutierten Genabschnitts über zwei neue *Bst*XI-Restriktionsschnittstellen an diesen Positionen zu erleichtern. Ebenso betrifft die vorliegende Erfindung die gezielte Einführung von Mutationen innerhalb oder außerhalb der vier Peptidschleifen, um bestimmte Eigenschaften des Lipocalinmuteins zu verbessern, z. B. seine Faltungsstabilität oder -effizienz oder seine Widerstandsfähigkeit gegenüber Proteasen. So kann beispielsweise durch den Aminosäureaustausch Lys87 zu Ser eine Spaltung des Bbp in zwei Fragmente, die ansonsten bei dessen Produktion in *E. coli* auftritt, unterdrückt werden.

Eine Oligomerisierung des ursprünglichen Bbp kann zudem durch die Mutation Asnl zu Asp vermieden werden. Auch kann durch den Austausch Cys116 zu Ser im ApoD dessen kovalente Quervernetzung mit anderen Proteinen verhindert und seine monomere Struktur stabilisiert werden.

In einer bevorzugten Ausführung der Erfindung dient dementsprechend die Bbp-Variante mit der Substitution Lys87 zu Ser als Grundstruktur zur Herstellung von Lipocalinmuteinen. Besonders bevorzugt wird die Bbp-Variante mit den Substitutionen Asn1 zu Asp, Asn21 zu Gln, Lys135 zu Met und Lys87 zu Ser zur Herstellung von Lipocalinmuteinen eingesetzt.

Auch längere Sequenzabschnitte innerhalb des für das Lipocalin kodierenden Gens können mittels bekannter Methoden einer Zufallsmutagenese unterworfen werden, z. B. durch Einsatz der Polyinerase-Kettenreaktion unter Bedingungen erhöhter Fehlerrate, durch chemische Mutagenese oder durch Verwendung bakterieller Mutatorstämme (Low et al., J. Mol. Biol. 260 (1996), 359-368). Derartige Methoden lassen sich auch zur weiteren Optimierung der Ligandenaffinität oder -spezifität eines bereits hergestellten Lipocalinmuteins verwenden. Mutationen, die dabei möglicherweise außerhalb der vier Schleifenregionen auftreten, können oft toleriert werden oder sich sogar als günstig erweisen, wenn sie z. B. zu einer verbesserten Faltungseffizienz oder -stabilität des Lipocalimuteins beitragen.

Nachdem die der Mutagenese unterzogenen kodierenden Nukleinsäuresequenzen zur Expression gebracht worden sind, können aus den verschiedenen Klonen der erhaltenen Bibliothek diejenigen Klone selektiert werden, die die genetische Information für Lipocalinmuteine tragen, welche einen vorgegebenen Liganden binden. Zur Selektion dieser Klone können bekannte Expressionsstrategien und Selektionsstrategien eingesetzt werden. Derartige Methoden sind beispielsweise im Zusammenhang mit der Herstellung oder dem Engineering rekombinanter Antikörperfragmente beschrieben worden, wie die "Phage Display"-Technik oder "Colony Screening"-Methoden (Skerra et al., Anal. Biochem. 196 (1991), 151-155).

Als Beispiel für ein erfindungsgemäßes Selektionsverfahren für Lipocalinmuteine mit den gewünschten Bindungseigenschaften sei hier eine Ausführungsform der "Phage Display"-Technik (Hoess, supra; Wells and Lowman, supra; Kay et al., Phage Display of Peptides and Proteins - A Laboratory Manual (1996), Academic Press) genannt. Die verschiedenen anderen möglichen Ausführungsformen der "Phage Display"-Technik werden hiermit per Referenz in die Offenbarung einbezogen. Für das beispielhafte Selektionsverfahren werden Phasmide hergestellt, welche die Expression des mutierten Lipocalin-Strukturgens als Fusionsprotein mit einer Signalsequenz am N-Terminus, bevorzugt der OmpA-Signalsequenz, und mit dem Hüllprotein pIII des Phagen M13 (Model und Russel, in "The Bacteriophages", Vol. 2 (1988), Plenum Press, New York, 375-456) oder Fragmenten dieses Hüllproteins, welche in die Phagenhülle eingebaut werden, am C-Terminus bewirken. Bevorzugt wird das C-terminale Fragment ΔpIII des Phagen-Hüllproteins, welches lediglich die Aminosäuren 217 bis 406 des natürlichen Hüllproteins pIII enthält, zur Herstellung der Fusionsproteine verwendet. Besonders bevorzugt wird ein C-terminales Fragment von pIII, in dem der Cysteinrest an der Position 201 fehlt oder durch eine andere Aminosäure ersetzt ist.

Das Fusionsprotein kann noch weitere Bestandteile enthalten, wie z. B. ein Affinitätsanhängsel oder eine Epitopsequenz für einen Antikörper, die den Nachweis, die Immobilisierung oder die spätere Reinigung des Fusionsproteins oder seiner Teile gestattet. Ferner kann sich zwischen der für das Lipocalin oder Lipocalinmuteine kodierenden Region und dem Genabschnitt für das Hüllprotein oder sein Fragment ein Stopcodon, vorzugsweise ein Amber-Stopcodon, befinden, das in einem geeigneten Suppressorstamm bei der Translation zumindest teilweise in eine Aminosäure übersetzt wird.

Als Phasmide werden hier bakterielle Plasmide bezeichnet, die die intergenische Region eines filamentösen Bakteriophagen, wie z. B. M13 oder f1 (Beck und Zink, Gene 16 (1981), 35-58) oder einen funktionellen Teil davon tragen, so daß bei Superinfektion der Bakterienzelle mit einem Helferphagen, beispielsweise M13K07, VCS-M13 oder R408, ein Strang der zirkulären Phasmid-DNA mit Hüllproteinen verpackt und als sogenanntes Phagemid in das Medium ausgeschleust wird. Dieses Phagemid hat einerseits das von dem jeweiligen Phasmid kodierte Lipocalinmutein als Fusion mit dem Hüllprotein pIII oder dessen Fragment an seiner Oberfläche eingebaut, wobei die Signalsequenz von dem Fusionsprotein in der Regel abgespalten wird. Andererseits trägt es eine oder mehrere Kopien des nativen Hüllproteins pIII von dem Helferphagen und ist somit in der Lage, einen Rezipienten - im allgemeinen einen Bakterienstamm, der ein F- oder F'-Plasmid trägt - zu infizieren. Auf diese Weise wird eine physikalische Kopplung zwischen der verpackten Nukleinsäure, die die genetische Information für das jeweilige Lipocalinmutein oder Lipocalinmutein trägt, und dem kodierten Protein gewährleistet, das zumindest teilweise in funktioneller Form an der Oberfläche des Phagemids präsentiert wird.

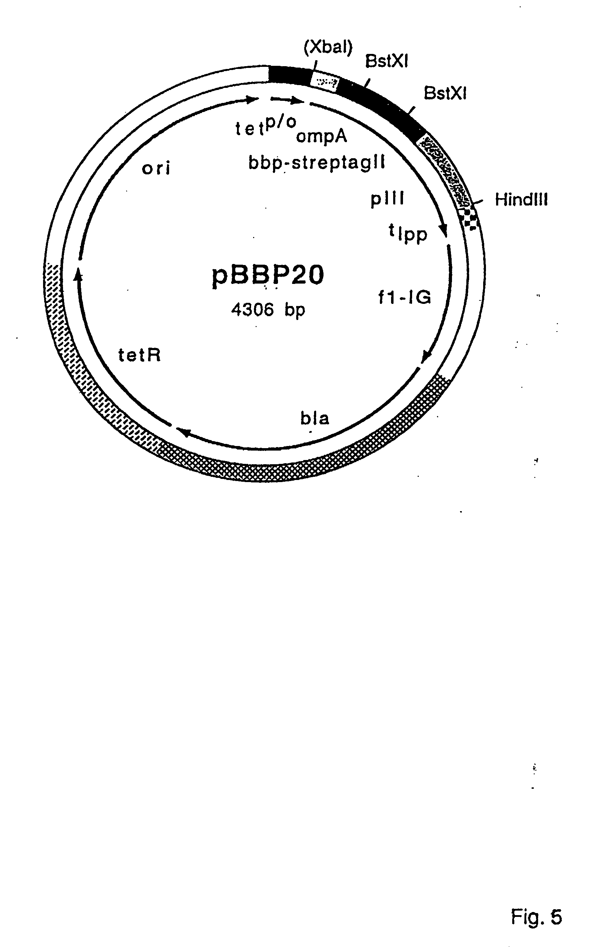

Zur Konstruktion der Phasmide mit den für die Bbp-Muteine kodierenden Sequenzen kann beispielsweise der Vektor pBBP20 (Figur 5) verwendet werden. Zur Selektion von Lipocalimutein ausgehend von einem anderen Lipocalin wird ein analoger Vektor hergestellt, indem die DNA-Sequenz, die für dieses Lipocalin oder seine Muteine kodiert, anstelle der für das Bbp kodierenden Sequenz in den Vektor pBBP20 inseriert wird. Im Fall des Bbp oder seiner Muteine kann die für die vier Peptidschleifen kodierende Nukleinsäure beispielsweise über die beiden BstXI-Restriktionsschnittstellen in den Vektor pBBP20 inseriert werden. Rekombinante Phasmide werden durch Transformation in den E. coli-Stamm, beispielsweise XL1-Blue (Bullock et al., BioTechniques 5 (1987), 376-379) oder TG1, eingebracht. Auf diese Weise werden Klone hergestellt, die zahlreiche verschiedene Lipocalinmuteine als Fusionsproteine produzieren können.

Anschließend wird diese Bibliothek, d. h. die Sammlung der erhaltenen Klone, nach bekannten Verfahren in Flüssigkultur mit einem M13-Helferphagen superinfiziert. Nach dieser Infektion kann die Inkubationstemperatur der Kultur zur Produktion der Phagemide abgesenkt werden. Bevorzugt werden Inkubationstemperaturen, bei denen eine optimale Faltung der Lipocalinmuteine als Bestandteil des Fusionsproteins mit dem Phagenhüllprotein oder seinem Fragment zu erwarten ist. Während oder nach der Infektionsphase kann in den Bakterienzellen die Expression des Gens für das Fusionsprotein mit dem Lipocalinmutein induziert werden. Die Induktionsbedingungen werden so gewählt, daß ein erheblicher Teil der produzierten Phagemide mindestens ein Lipocalinmutein präsentiert. Die Phagemide werden nach einer Inkubationsphase der Kultur von beispielsweise 6 bis 8 h isoliert. Zur Isolierung der Phagemide sind verschiedene Verfahren, wie z. B. die Präzipitation mit Polyethylenglykol bekannt.

Die isolierten Phagemide können durch Inkubation mit dem gewünschten Liganden einer Selektion unterworfen werden, wobei der Ligand in einer Form vorliegt, die eine zumindest vorübergehende Immobilisierung derjenigen Phagemide ermöglicht, die Lipocalinmuteine mit der gewünschten Bindungsaktivität als Fusionsprotein in ihrer Hülle tragen. Unter den verschiedenen dem Fachmann bekannten Ausführungsformen kann der Ligand beispielsweise mit einem Trägerprotein, wie Serumalbumin, konjugiert und über dieses Trägerprotein an eine proteinbindende Oberfläche, beispielsweise Polystyrol, gebunden werden. zu dieser Immobilisierung des Liganden lassen sich bevorzugt die für ELISA-Techniken geeigneten Mikrotiterplatten oder sogenannte "Immuno-Sticks" verwenden. Alternativ können auch Konjugate des Liganden mit anderen bindefähigen Gruppen, wie z. B. Biotin, eingesetzt werden. Der Ligand läßt sich dann an Oberflächen immobilisieren, die diese Gruppe selektiv binden, wie z. B. mit Streptavidin oder Avidin beschichtete Mikrotiterplatten oder paramagnetische Partikel.

Vorhandene Proteinbindungsstellen an den mit dem Liganden besetzten Oberflächen können mit den für ELISA-Verfahren bekannten Blockierungslösungen abgesättigt werden. Anschließend werden die Phagemide beispielsweise in einem physiologischen Puffer mit dem an der Oberfläche immobilisierten Liganden in Kontakt gebracht. Ungebundene Phagemide werden durch mehrfaches Waschen entfernt. Die an der Oberfläche verbleibenden Phagemidpartikel werden anschließend eluiert. Zur Elution kann der freie Ligand in gelöster Form zugegeben werden. Die Phagemide können aber auch durch Zugabe von Proteasen oder unter mäßig denaturierenden Bedingungen, z. B. in Gegenwart von Säuren, Laugen, Detergentien oder chaotropen Salzen, eluiert werden. Eine bevorzugte Methode ist die Elution mittels Puffern mit pH 2,2, wobei das Eluat anschließend neutralisiert wird.

Danach werden E. coli-Zellen mittels allgemein bekannter Methoden mit den eluierten Phagemiden infiziert. Die Nukleinsäure kann auch aus den eluierten Phagemiden extrahiert und auf andere Weise in die Zellen eingebracht werden. Ausgehend von den dabei erhaltenen E. coli-Klonen werden durch Superinfektion mit M13-Helferphagen nach dem oben beschriebenen Verfahren wiederum Phagemide erzeugt und die auf diese Weise vermehrten Phagemide erneut einer Selektion an der Oberfläche mit dem immobilisierten Liganden unterworfen. Oft sind mehrere Selektionszyklen notwendig, um die Phagemide mit den Lipocalinmuteinen in angereicherter Form zu erhalten. Die Anzahl der Selektionszyklen wird bevorzugt so gewählt, daß bei der anschließenden funktionellen Analyse mindestens 0,1% der untersuchten Klone Lipocalinmuteine mit nachweisbarer oder bestimmbarer Affinität zu dem vorgegebenen Liganden produzieren. Abhängig vom Umfang, d. h. der Komplexität der eingesetzten Bibliothek sind dazu typischerweise 2 bis 8 Zyklen notwendig.

Zur funktionellen Analyse der selektierten Muteine wird ein E. coli-Stamm mit den nach den Selektionszyklen erhaltenen Phagemiden infiziert und die entsprechende doppelsträngige Phasmid-DNA isoliert. Ausgehend von dieser Phasmid-DNA oder auch von der aus den Phasmiden extrahierten einzelsträngigen DNA kann die Nukleinsäuresequenz der selektierten Lipocalinmuteine mittels der dazu üblichen Methoden bestimmt und die Aminosäuresequenz daraus abgeleitet werden. Die mutierte Region oder die Sequenz des gesamten Lipocalinmuteins oder Lipocalinmuteins kann in einem anderen Expressionsvektor subkloniert und in einem geeigneten Wirtsorganismus exprimiert werden. Als Expressionsvektor kann beispielsweise pBBP21 verwendet werden, und die Expression mit pBBP21-Derivaten kann in E. coli-Stämmen, beispielsweise E. coli-TG1, durchgeführt werden. Die gentechnisch hergestellten Lipocalinmuteine können durch verschiedene proteinchemische Verfahren gereinigt werden. Die beispielsweise mit pBBP21 produzierten Anticaline tragen das Affinitätspeptid Strep-Tag II (Schmidt et al., J. Mol. Biol. 255 (1996), 753-766) an ihrem C-Terminus und können daher bevorzugt mittels der Streptavidin-Affinitätschromatographie gereinigt werden.

Die Selektion kann ebenso mittels anderer Methoden durchgeführt werden. Eine Vielzahl entsprechender Ausführungsformen ist dem Fachmann bekannt oder in der Literatur beschrieben. Auch eine Kombination von Methoden kann angewandt werden. Beispielsweise können Klone, die durch "Phage Display" selektiert oder zumindest angereichert wurden, zusätzlich einem "Colony Screening" unterzogen werden. Diese Vorgehensweise hat den Vorteil, daß dabei direkt einzelne Klone hinsichtlich der Produktion von Lipocalinmuteinen mit nachweisbarer Bindungsaffinität für einen Liganden isoliert werden können.

Neben der Verwendung von E. coli als Wirtsorganismus bei der "Phage Display"-Technik oder der "Colony Screening"-Methode lassen sich beispielsweise auch andere Bakterienstämme, Hefen oder auch Insekten- oder Säugerzellen dazu heranziehen. Zusätzlich zur Selektion eines Lipocalinmuteins aus einer primären Bibliothek, die ausgehend von einer kodierenden Nukleinsäuresequenz für ein Lipocalin hergestellt wurde, können vergleichbare Methoden auch angewandt werden, um ein Lipocalinmutein durch wiederholte, ggf. eingeschränkte Mutagenese seiner kodierenden Nukleinsäuresequenz hinsichtlich Affinität oder Spezifität für den gewünschten Liganden zu optimieren.

Es ist überraschend, daß mit dem erfindungsgemäßen Verfahren Anticaline gewonnen werden können, die hohe Affinität zu einem vorgegebenen Liganden zeigen. Mit den in den Beispielen beschriebenen Lipocalinmuteinen wurden für verschiedene Fluoresceinderivate Bindungskonstanten bestimmt, die mehr als $10^6$ M$^{-1}$ betrugen. Diese Affinitätswerte liegen in derselben Größenordnung wie die Affinitäten der Lipocaline zu ihren natürlichen Liganden, beispielsweise von Rbp zu Vitamin A (Cogan et al., Eur. J. Biochem. 65 (1976), 71-78). Im Gegensatz zu den natürlichen Liganden der Lipocaline, die in der Regel wasserunlöslich und chemisch unbeständig sind, handelt es sich allerdings bei Fluorescein um eine relativ hydrophile Verbindung, die auch in immunologischen Studien als Hapten mit Modellcharakter eingesetzt wurde (Voss, Fluorescein Hapten: An Immunological Probe (1984), CRC Press). Zudem zeigt das Fluorescein mit dem Biliverdin IX$\gamma$, dem ursprünglichen Liganden des Bbp, keinerlei strukturelle Verwandtschaft.

Solche mit den Lipocalinmuteinen erzielbare Affinitäten für neue Liganden sind vergleichbar mit den Affinitäten, welche für Antikörper aus der sekundären Immunantwort bekannt sind. Darüber hinaus besteht zusätzlich die Möglichkeit, die hergestellten Lipocalinmuteine einer weiteren, ggf. partiellen Zufallsmutagenese zu unterwerfen, um aus der dabei erhaltenen neuen Bibliothek Varianten mit noch höherer Affinität zu selektieren. Entsprechende Vorgehensweisen wurden bereits im Fall rekombinanter Antikörperfragmente zum Zweck einer "Affinitätsmaturierung" beschrieben (Low et al., supra; Barbas und Burton, Trends Biotechnol. 14 (1996), 230-234) und lassen sich vom Fachmann auch auf die Lipocalinmuteine in entsprechender Weise anwenden.

Überraschenderweise zeigte sich weiterhin, daß die vier Peptidschleifen, welche die Liganden-Bindungstasche der Lipocaline bilden, hohe Toleranz für Aminosäuresubstitutionen aufweisen, ohne daß die Faltung der Polypeptidkette in den gewonnenen Lipocalinmuteinen dadurch wesentlich beeinträchtigt wird. Dementsprechend ist es möglich, Lipocalinmuteine zu generieren, die Bindungstaschen mit vielfältigen Oberfächeneigenschaften aufweisen, so daß die molekulare Erkennung von unterschiedlichsten Liganden, auch von Peptiden oder Polypeptiden sowie anderen Makromolekülen, realisiert werden kann.

Ist die genetische Information für ein erst einmal vorhanden oder seine Aminosäuresequenz bekannt, so läßt es sich mit allgemein bekannten gentechnischen Verfahren produzieren. Bevorzugt sind Verfahren zur Herstellung von Lipocalinmuteinen, wobei das Lipocalinmutein, ein Fragment des Lipocalinmutein oder ein Fusionsprotein aus dem Lipocalinmutein und einem anderen Polypeptid ausgehend von der für das Lipocalinmutein kodierenden Nukleinsäure mittels gentechnischer Methoden in einem bakteriellen oder eukaryontischen Wirtsorganismus produziert und aus diesem Wirtsorganismus oder dessen Kultur gewonnen wird. Die Tatsache, daß dabei in der Regel nur ein Strukturgen zur Expression gebracht werden muß, stellt eine erhebliche Vereinfachung im Vergleich zu den Antikörpern oder ihren Fragmenten dar.

Eine Vielzahl von Wirtsorganismen, wie *E. coli* und andere Gram-negative oder auch Gram-positive Bakterien, Hefen und andere eukaryontische Zellen, kann zur gentechnischen Herstellung eingesetzt werden. Auch die Wahl zwischen diversen Expressionsstrategien ist möglich. So führt beispielsweise im Wirtsorganismus *E. coli* die Sekretion mit einer geeigneten Signalsequenz, wie in den Beispielen beschrieben, zum korrekt gefalteten, funktionellen Protein, in dem die Disulfidbindungen ausgebildet sind. Andererseits ist es ebenfalls möglich, ein Lipocalinmutein im Cytosol einer Bakterienzelle zu produzieren und, falls das Lipocalin im Cytosol nicht funktionell gefaltet wird, dieses erst *in vitro* funktionell zu falten. Selbst eine Faltung aus Aggregaten, welche sich bei der Sekretion ggf. im Periplasma des Bakteriums ansammeln, ist möglich.

Gentechnisch hergestellte Lipocalinmuteine mittels einer Vielzahl etablierter Methoden gereinigt werden. Die Eignung der Methode hängt jeweils vom verwendeten Wirtsorganismus, der Expressionsstrategie und anderen Faktoren ab, die dem in der Expression und Reinigung rekombinanter Proteine erfahrenen Fachmann bekannt sind. Die Reinigung kann ggf. vereinfacht werden, indem das Lipocalinmutein mit einer oder mehreren Peptidsequenzen fusioniert wird. Bevorzugt sind zur Fusion solche Peptide oder Proteine zu verwenden, die dem resultierenden rekombinanten Protein Affinität zu bestimmten Säulenmaterialien verleihen. Solche Fusionen sollten die Funktion des Lipocalinmuteins nicht negativ beeinflussen oder müssen z. B. durch Einfügung geeigneter Proteaseschnittstellen abspaltbar sein. Als typische Beispiele für Fusionspartner seien Oligohistidin-Anhängsel, das Strep-Tag oder das Strep-Tag II, die Glutathion-S-Transferase, das Maltose-Bindungsprotein oder die Albumin-Bindungsdomäne des Protein G genannt. Ebenso können Lipocalimuteine über ihre jeweilige Liganden-Bindungsstelle mittels einer Affinitätschromatographie an dem an einer Säulenmatrix immobilisierten zugehörigen Liganden, bzw. geeigneten Derivaten dieses Liganden, gereinigt werden. Der Aufbau der Anticaline aus einer einzelnen Polypeptidkette ist im Vergleich mit rekombinanten Antikörperfragmenten bei der Reinigung von Vorteil, da keine Maßnahmen ergriffen werden müssen, um die intakte Assoziation von Untereinheiten zu gewährleisten.

Die Struktur eines Lipocalinmuteins kann zum Zweck der verbesserten Produktion, Reinigung oder Anwendbarkeit zusätzlich modifiziert werden. So kann beispielsweise das N- oder das C-terminale Peptidsegment, welches nicht Bestandteil der β-Barrel-Struktur ist, entfernt werden. Vorhandene Disulfidbindungen können durch Substitution der Cysteinreste eliminiert, oder neue Disulfidbindungen können an anderer Stelle eingeführt werden. Freie Cysteinreste, wie der Rest 116 im ApoD, können entfernt werden, wenn sie z. B. die Produktion oder die Stabilität des Lipocalinmuteins beeinträchtigen. Ggf. können auch Cysteinreste neu eingeführt werden, um z. B. entsprechende Proteinkonjugate durch chemische Kopplung mit anderen Komponenten herzustellen. Auch können außerhalb der eigentlichen Ligandenbindungstasche Bindungsstellen für weitere Liganden, wie z. B. Metallionen, in das Anticalin eingebaut werden. Schließlich können auch zu anderen Zwecken als der Proteinproduktion oder -reinigung Fusionsproteine aus Lipocalinmuteinen und anderen Polypeptiden, Proteinen oder Proteindomänen mittels dem Fachmann bekannter Methoden hergestellt werden. Die Fusion kann bevorzugt am N-Terminus oder auch am C-Terminus des Lipocalinmuteins erfolgten.

Der artige Fusionen können geeignet sein, um dem Lipocalinmutein neue Eigenschaften zu vermitteln, wie z. B. enzymatische Aktivität oder Affinität zu anderen Molekülen, wie Proteinen, Makromolekülen oder niedermolekularen Liganden. Beispielsweise sind Fusionen mit Enzymen, welche chromogene oder fluorogene Reaktionen katalysieren oder zur Freisetzung von cytotoxischen Agenzien dienen können, möglich. Weitere Beispiele für Fusionspartner, die in der Praxis von Vorteil sein können, sind Bindungsdomänen wie die Albumin-Bindungsdomäne von Protein G, Protein A, Antikörperfragmente, Oligomerisierungsdomänen, Toxine oder auch Lipocalinmuteine mit anderer oder derselben Ligandenspezifität. Alternativ zur Herstellung von Fusionsproteinen können auch Konjugate aus Lipocalinmuteinen und Proteinen, Nukleinsäuren oder nahezu beliebigen Biomolekülen und chemischen Verbindungen anhand dem Fachmann bekannter Methoden hergestellt werden.

Lipocalinmuteine und ihre Derivate können ähnlich wie die Antikörper oder deren Fragmente in vielen Bereichen eingesetzt werden. Bevorzugt werden Lipocalinmuteine verwendet zur Bindung an eine Festphase, so daß der Ligand des Lipocalinmuteins oder ein Konjugat oder Fusionsprotein dieses Liganden immobilisiert oder abgetrennt werden kann. Weiterhin bevorzugt ist die Verwendung von Lipocalinmuteinen zur Markierung mit einem Enzym, einem Antikörper, einer radioaktiven Substanz oder einer anderen Gruppe mit einer biochemischen Aktivität oder mit definierten Bindungseigenschaften, so daß der Ligand des Lipocalinmuteins oder ein Konjugat oder Fusionsprotein dieses Liganden damit nachgewiesen oder in Kontakt gebracht werden kann. Lipocalinmuteine können beispielsweise zum Nachweis chemischer Strukturen mittels etablierter bioanalytischer Methoden wie ELISA oder Westernblot, in der Mikroskopie oder in der Immunsensorik dienen. Das Nachweissignal kann dabei entweder direkt unter Einsatz eines geeigneten LipocalinmuteinKonjugats oder - fusionsproteins erzeugt werden oder indirekt durch Detektion des gebundenen Lipocalinmuteins mittels eines dagegen gerichteten Antikörpers oder z. B. unter Verwendung eines Affinitätsanhängsels.

Bevorzugte Liganden für Lipocalinmuteine sind einerseits chemische Verbindungen in freier oder konjugierter Form, die Merkmale eines immunologischen Haptens aufweisen, und andererseits Peptide, Polypeptide oder andere Makromoleküle wie auch entsprechende Konjugate davon. Ein interessantes Anwendungsgebiet ist der Einsatz der Lipocalinmuteine zum Zweck des Nachweises von nicht radioaktiv markierten Biomolekülen, insbesondere Nukleinsäuren. So sind zum Beispiel chemisch reaktive Derivate des Fluoresceins zur Markierung von Proteinen oder von Nukleinsäuren kommerziell verfügbar, und auch Verfahren zum Einbau von Fluoresceingruppen bei der Synthese oder Replikation von Nukleinsäuren sind bekannt. Entsprechend modifizierte Nukleinsäuren lassen sich als spezifische Gensonden verwenden und anschließend mit den in den Beispielen beschriebenen Lipocalinmuteinen

Lipocalinmuteine können auch einen Löschungseffekt auf die Fluoreszenz des von ihnen gebundenen Liganden, wie z. B. Fluorescein, ausüben. Für solche Lipocalinmuteine ergeben sich vielversprechende Einsatzmöglichkeiten bei biophysikalischen Untersuchungen. So läßt sich der Fluoreszenz-Löschungseffekt eines Lipocalinmuteins beispielsweise ähnlich wie bei bestimmten Antikörpern gegen Fluorescein ausnutzen, um die Orientierung eines mit Fluorescein markierten Membranproteins in der Lipid-Doppelschicht zu bestimmen. Ein weiterer Anwendungsbereich liegt in der Untersuchung der Dynamik von Ligand/Rezeptor-Wechselwirkungen auf der Zelloberfläche, wobei der Ligand mit einer Fluoresceingruppe markiert ist. Auch vielfältige andere Anwendungen, bei denen die Fluoreszenzlöschung von Fluorescein oder anderen fluoreszierenden Verbindungen eine Rolle spielt, sind möglich. Beispielsweise kann ein Lipocalinmutein eingesetzt werden, um eine störende Hintergrundintensität durch überschüssige fluoreszenzmarkierte Reagenzien in der Fluoreszenzmikroskopie von Zellen zu vermeiden.

Zahlreiche Anwendungsmöglichkeiten für die Lipocalinmuteinen liegen in der Medizin. Neben dem Einsatz in der Diagnostik können auch Lipocalinmuteinen hergestellt werden, welche beispielsweise gewebs- oder tumorspezifische zelluläre Oberflächenmoleküle binden. Entsprechende Lipocalinmuteinen können in konjugierter Form oder als Fusionsproteine zum "Tumor Imaging" oder direkt zur Krebstherapie eingesetzt werden. Zur Herstellung solcher Lipocalinmuteinen kann es zweckmäßig sein, von einem menschlichen Lipocalin auszugehen, wie z. B. dem Rbp oder dem ApoD. Die geringe Größe der Lipocalinmuteinen oder ihrer Derivate hat dabei gegenüber den Antikörpern neue und vorteilhafte Eigenschaften zur Folge.

Die Erfindung wird weiter veranschaulicht durch die nachstehenden Beispiele und die beigefügten Zeichnungen, in denen:

Figur 1 die molekulare Raumstruktur des Bbp mit seinem Liganden Biliverdin IXγ schematisch darstellt (A) und die räumliche Position derjenigen Aminosäuren angibt (B), die bevorzugt Gegenstand der Mutagenese zur Herstellung von Lipocalinmuteinen sind;

Figur 2 die Topographie der Polypeptidkette für die Liganden- Bindungsstellen von Antikörpern (A) und von Lipocalinen (B) miteinander vergleicht;

Figur 3 die Aminosäuresequenzen verschiedener Lipocaline gegenüberstellt;

Figur 4 die Herstellung der Bibliothek der Lipocalinmuteine auf der Ebene der Nukleinsäuren schematisch veranschaulicht;

Figur 5 den Phasmidvektor pBBP20 schematisch wiedergibt;

Figur 6 die Expressionsvektoren pBBP21 (A) und pBBP22 (B) schematisch darstellt;

Figur 7 die Bindung eines Peptids durch Lipocalinmuteine in einem ELISA demonstriert;

Figur 8 die Komplexierung und Fluoreszenzlöschung des Liganden Fluorescein durch ein Lipocalinmutein in einer Fluoreszenztitration wiedergibt.

Figur 1 zeigt die Kristallstruktur des Bbp (Datei 1BBP aus der Brookhaven Protein Data Bank; Molekül A), die mit Hilfe des Programms MOLSCRIPT (Kraulis, J. Appl. Cryst. 24 (1991), 946-950) graphisch dargestellt wurde. In (A) sind der gebundene Ligand wie auch die beiden Disulfidbindungen in dem Polypeptid als "Ball and Stick" wiedergegeben (Kohlenstoff: schwarz; Stickstoff, Schwefel: dunkelgrau; Sauerstoff: hellgrau). Die einzelnen β-Faltblattstränge sind als Bänder und die α-Helix ist als Spirale abgebildet. Die kelchartige Form der Liganden-Bindungsstelle ist oben am offenen Ende des aus den acht antiparallelen β-Faltblattsträngen gebildeten β-Barrels zu erkennen. In (B) sind die C$^\alpha$-Positionen der Aminosäuren als entlang der Polypeptidkette miteinander verbundene Kugeln wiedergegeben. Der N- und der C-Terminus des Polypeptids ist markiert. Die schwarz dargestellten C$^\alpha$-Positionen sind mit den Sequenznummern bezeichnet und geben die Lage der in den Beispielen mutierten Aminosäuren in der Struktur des Bbp an.

Figur 2 zeigt eine Aufsicht (A) auf die Antigen-Bindungsstelle im Fv-Fragment eines Immunglobulins, welche gemeinsam von den variablen Domänen VL und VH gebildet wird, und (B) auf die Liganden-Bindungsstelle eines Lipocalins. Die β-Faltblattstränge sind jeweils angenähert senkrecht zur Papierebene angeordnet und als Balken dargestellt. Die sechs CDRs (L1, L2, L3, H1, H2, H3) im Immunglobulin sowie die vier Peptidschleifen im Lipocalin verbinden jeweils zwei β-Faltblattstränge miteinander. Die anderen Verbindungssegmente und Strukturelemente sind weggelassen.

Figur 3 zeigt einen Sequenzvergleich (Angabe der Aminosäuren im Einbuchstaben-Code) zwischen dem Bilin-Bindungsprotein (SWISS-PROT Datenbank-Zugriffscode P09464), dem menschlichen Apolipoprotein D (SWISS-PROT Datenbank-Zugriffscode P05090) und dem Retinol-Bindungsprotein (SWISS-PROT Datenbank-zugriffscode P02753) in Form der maturen Polypeptide. Die acht Segmente im Bereich des β-Barrels, welche den konservierten β-Faltblattsträngen entsprechen und in den Kristallstrukturen von Bbp und Rbp große Ähnlichkeit aufweisen, sind durch Unterstreichung hervorgehoben. Die Schleifenregionen, in denen Aminosäuren bevorzugt ausgetauscht werden sollen, sind unterhalb der Sequenz des Bbp durch doppeltes Unterstreichen gekennzeichnet. Diejenigen Positionen im Bbp, welche in den Beispielen mutiert wurden, sind zusätzlich durch Sterne markiert. Das Alignment zwischen den Sequenzen von Bbp und ApoD entspricht derjenigen in der Publikation von Peitsch und Boguski (New Biologist 2 (1990), 197-206).

Figur 4 zeigt schematisch eine Strategie zur konzertierten Mutagenese von 16 ausgewählten Aminosäurepositionen im Bbp durch wiederholte Anwendung der Polymerase-Kettenreaktion (PCR). Für jede der vier Peptidschleifen des Lipocalins, in der Aminosäuren mutiert werden sollten, wurde ein Oligodesoxynukleotid synthetisiert, SEQ ID NO: 1, SEQ ID NO:2, SEQ ID NO:3 und SEQ ID NO:4, wobei an den Mutationsstellen jeweils die im Sequenzprotokoll angegebenen Mischungen der Basenbausteine eingesetzt wurden. Aufgrund der gewählten Zusammensetzung konnte an allen mutierten Codons aus den drei insgesamt möglichen Stopcodons ggf. nur das Amber-Stopcodon, TAG, entstehen, welches in den zur Genexpression verwendeten *E. coli sup*E-Stämmen XL1-Blue oder TG1 als Glutamin translatiert wird. Für bestimmte Anwendungen, beispielsweise zur Genexpression in anderen Bakterienstämmen oder Organismen, kann ein solches Nonsense-Codon, wenn es im Strukturgen für ein selektiertes Lipocalinmutein auftritt, vom Fachmann z. B. mittels ortsgerichteter Mutagenese durch ein für Glutamin kodierendes Codon substituiert werden. Mit den Primern SEQ ID NO:1 und SEQ ID NO:2 wurde unter Verwendung der pBBP20-Plasmid-DNA (SEQ ID NO:10), die das Bbp-Strukturgen enthält, als Matrize ein Nukleinsäurefragment mit 159 Basenpaaren amplifiziert (1. Schritt, A). Parallel dazu wurde mit den Primern SEQ ID NO:3 und SEQ ID NO:4, ebenfalls unter Verwendung von pBBP20 als Matrize, ein Nukleinsäurefragment mit 164 Basenpaaren amplifiziert (1. Schritt, B). Die Mischung dieser beiden Fragmente diente als Matrize in einem 2. Amplifizierungsschritt in Gegenwart eines mit den beiden Fragmenten hybridisierenden Oligodesoxynukleotids SEQ ID NO:5 sowie der beiden flankierenden PCR-Primer SEQ ID NO:6 und SEQ ID NO:7, wobei ein Genfragment von 371 Basenpaaren erhalten wurde. Dieses enthielt alle 16 mutierten Codons und wurde anschließend mittels der beiden *Bst*XI-Schnittstellen in dem Vektor pBBP20 kloniert. Die Verwendung dieser beiden Restriktionsschnittstellen, die durch ihre spezielle Anordnung beim Restriktionsverdau zu zwei nicht kompatiblen überhängenden DNA-Enden führten, ermöglichte eine besonders effiziente Ligierung. Zur Einführung der beiden *Bst*XI-Schnittstellen in das Bbp-Strukturgen waren zuvor die beiden Aminosäuresubstitutionen Asn21 zu Gln und Lys135 zu Met gegenüber der ursprünglichen Sequenz vorgenommen worden.

Figur 5 zeigt eine Zeichnung von pBBP20. Dieser Vektor kodiert für ein Fusionsprotein aus der OmpA-Signalsequenz, einem veränderten Bbp mit den vier Aminosäuresubstitutionen AsnI zu Asp, Asn21 zu Gln, Lys87 zu Ser und Lys135 zu Met, dem Strep-Tag II-Affinitätsanhängsel und einer verkürzten Form des Hüllproteins pIII von M13, umfassend die Aminosäuren 217 bis 406 (pIII). Das Strukturgen steht unter der Transkriptionskontrolle des Tetracyclin-Promotor/Operators (tet$^{p/o}$) und endet am Lipoprotein-Transkriptionsterminator (t$_{lpp}$). Weitere Elemente des Vektors sind der Replikationsursprung (ori), die intergenische Region des filamentösen Bakteriophagen f1 (f1-IG), das für die β-Lactamase kodierende Ampicillin-Resistenzgen (bla) und das Tetracyclin-Repressorgen (tetR). Zwischen der kodierenden Region für das Bbp mit der OmpA-Signalsequenz und dem Strep-Tag II sowie der kodierenden Region für das verkürzte Phagenhüllprotein pIII befindet sich ein Amber-Stopcodon, welches in einem Amber-Suppressor-Wirtsstamm teilweise überlesen wird. Die beiden *Bst*XI-Schnittstellen, die zur Klonierung der mutierten Genkassette verwendet wurden, und die das Strukturgen flankierenden Restriktionsschnittstellen sind markiert. Ein relevanter Ausschnitt aus der Nukleinsäuresequenz von pBBP20 ist mit der kodierten Aminosäuresequenz im Sequenzprotokoll als SEQ ID NO:10 wiedergegeben. Der Ausschnitt beginnt mit einer Hexanukleotidsequenz, die durch Ligierung eines *Xba*I-Überhangs mit einem dazu komplementären *Spe*I-Überhang erhalten wurde, und endet mit der *Hind*III-Schnittstelle. Die Vektorelemente außerhalb dieses Bereichs sind identisch mit dem Vektor pASK75, dessen vollständige Nukleotidsequenz in der Offenlegungsschrift DE 44 17 598 A1 angegeben ist.

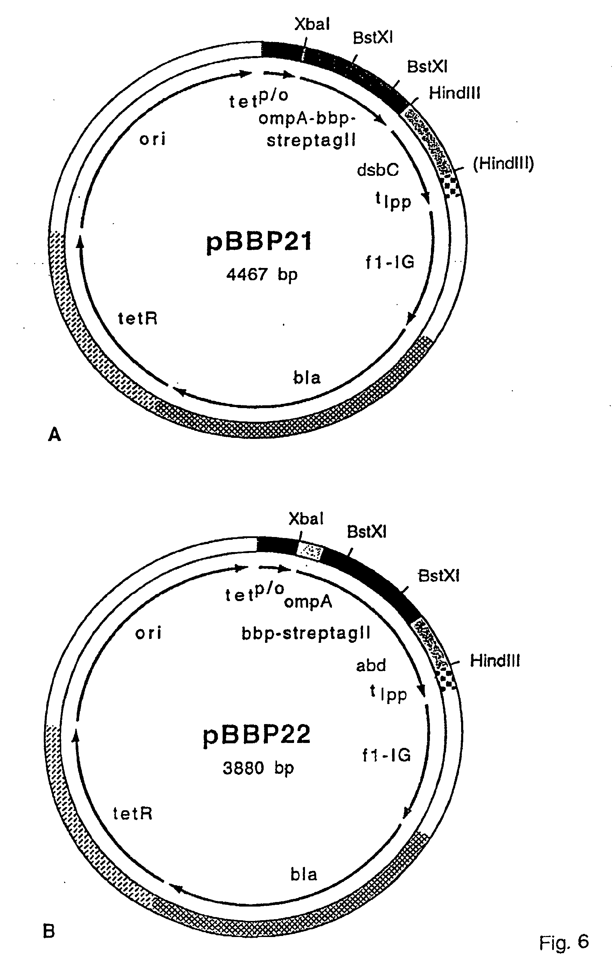

Figur 6 zeigt eine Zeichnung von pBBP21 (A) und von pBBP22 (B). pBBP21 kodiert für ein Fusionsprotein aus der OmpA-Signalsequenz, einem veränderten Bbp gemäß Figur 5 und dem Strep-Tag II-Affinitätsanhängsel. Dieses Strukturgen wird von dem *dsb*C-Strukturgen (einschließlich dessen ribosomaler Bindungsstelle) aus *E. coli* (Zapun et al., Biochemistry 34 (1995), 5075-5089) als einem zweiten Cistron gefolgt. Das dadurch gebildete künstliche Operon steht unter gemeinsamer Transkriptionskontrolle des Tetracyclin-Promotor/Operators (tet$^{p/o}$) und endet am Lipoprotein-Transkriptionsterminator (t$_{lpp}$). Alle weiteren genetischen Elemente sind identisch mit pBBP20 gemäß Figur 5. Die mit der Kosekretion verbundene Überproduktion der bakteriellen Disulfidisomerase DsbC kann die Knüpfung der richtigen Disulfidbrücken in dem Lipocalin unterstützen und so die Ausbeute an korrekt gefaltetem Polypeptid steigern. Allerdings ist die Produktion des Lipocalins oder der Lipocalimuteine auch ohne diese Maßnahme möglich. Ein relevanter Ausschnitt aus der Nukleinsäuresequenz von pBBP21 ist mit der kodierten Aminosäuresequenz im Sequenzprotokoll als SEQ ID NO:11 wiedergegeben. Der Ausschnitt beginnt mit der *Xba*I-Schnittstelle und endet mit einem Hexanukleotid, das durch Ligierung eines stumpfen Strangendes mit einem aufgefüllten *Hind*III-Strangende erhalten wurde, wobei die ursprüngliche *Hind*III-Schnittstelle verloren ging. Die Vektorelemente außerhalb dieses Bereichs sind identisch mit dem Vektor pASK75, dessen vollständige Nukleotidsequenz in der Offenlegungsschrift DE 44 17 598 A1 angegeben ist. pBBP22 kodiert für ein Fusionsprotein aus der OmpA-Signalsequenz, einem veränderten Bbp gemäß Figur 5, dem Strep-Tag II-Affinitätsanhängsel und einer Albumin-Bindungsdomäne (abd) des Protein G aus *Streptococcus* (Kraulis et al., FEBS Lett. 378 (1996), 190-194). Alle weiteren genetischen Elemente sind identisch mit pBBP20. Ein relevanter Ausschnitt aus der Nukleinsäuresequenz von pBBP22 ist mit der kodierten Aminosäuresequenz im Sequenzprotokoll als SEQ ID NO:12 wiedergegeben. Der Ausschnitt beginnt mit der *Xba*I-Schnittstelle und endet mit der *Hind*III-Schnittstelle. Die Vektorelemente außerhalb dieses Bereichs sind identisch mit dem Vektor pASK75, dessen vollständige Nukleotidsequenz in der Offenlegungsschrift DE 44 17 598 A1 angegeben ist.

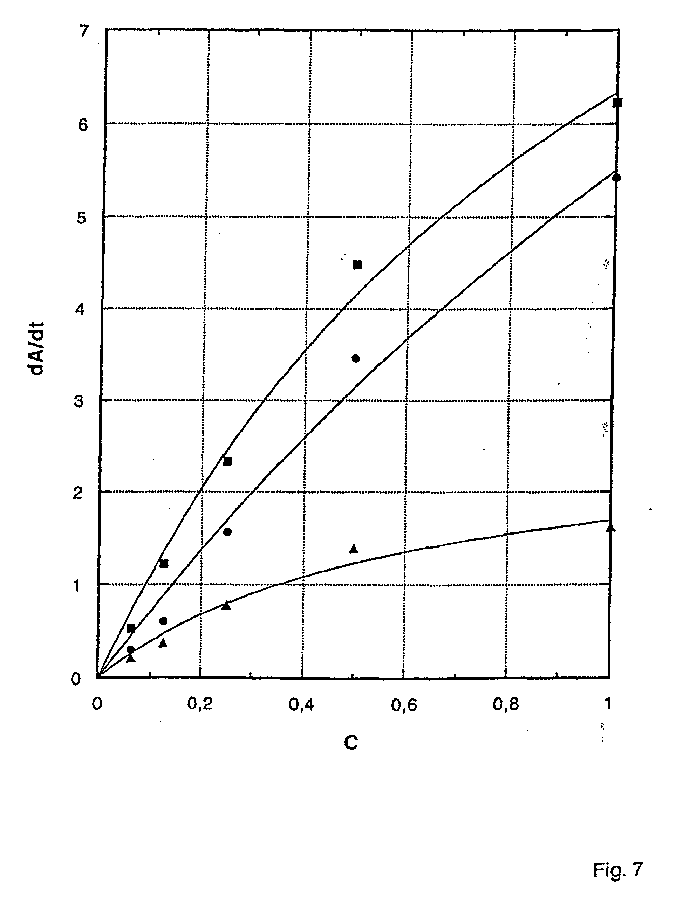

Figur 7 zeigt eine graphische Darstellung der Daten aus Beispiel 7, in der ein synthetisiertes Peptidepitop des Hepatitis C-Virus mit den Lipocalinmuteinen HepC1 (Quadrate) und HepC4 (Kreise) in einem ELISA nachgewiesen wurde. Zum Vergleich sind die mit dem Bbp (Dreiecke) erhaltenen Werte aufgetragen. "C" steht für die relative Proteinkonzentration innerhalb jeder Verdünnungsreihe.

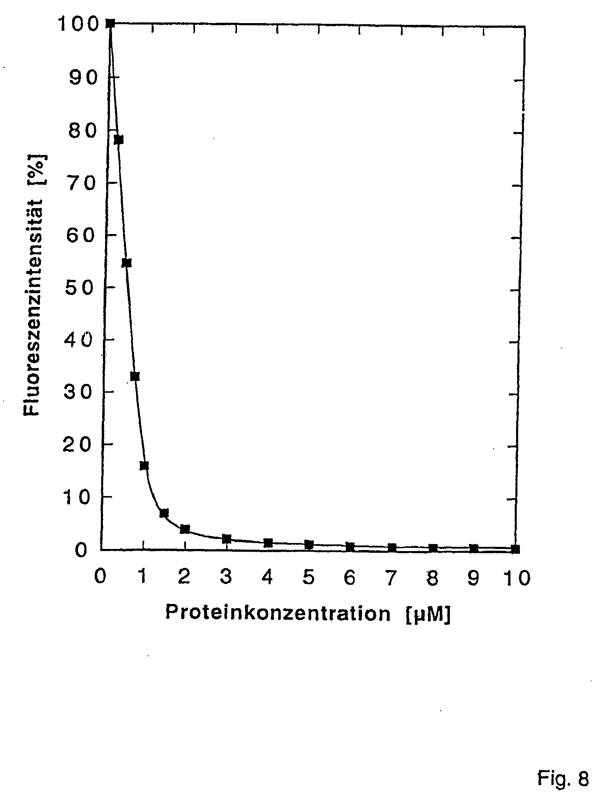

Figur 8 zeigt eine graphische Darstellung der Daten aus Beispiel 8, bei der eine 1 μM Lösung von Fluorescein mit unterschiedlichen Konzentrationen des Lipocalinmuteins FluA versetzt wurde. Die Fluoreszenzintensitäten wurden bei einer Anregungswellenlänge von 490 nm und einer Emissionswellenlänge von 512 nm gemessen und gegen die jeweilige Gesamtkonzentration des Lipocalinmuteins im Ansatz aufgetragen. Die Datenpunkte wurden schließlich durch eine Ausgleichskurve angepaßt.

**Beispiele**

**Beispiel 1: Herstellung einer Bibliothek für Lipocalinmuteine**

Sofern nicht anders angegeben, wurden die dem Fachmann geläufigen gentechnischen Methoden, wie sie z. B. in Sambrook et al. (Molecular Cloning. A Laboratory Manual (1989), Cold Spring Harbor Press) beschrieben sind, verwendet.

Zur konzertierten Mutagenese von insgesamt 16 ausgewählten Aminosäurepositionen in den vier Peptidschleifen des Bbp wurde die PCR in mehreren Schritten gemäß Figur 4 angewandt. Die PCR-Reaktionen wurden in den ersten beiden Amplifizierungsschritten in einem Volumen von 50 $\mu$l durchgeführt, wobei 10 ng pBBP20-Plasmid-DNA als Matrize sowie jeweils 25 pmol der Primer, welche nach der üblichen Phosphoramidit-Methode synthetisiert worden waren, eingesetzt wurden. Zudem enthielt der Reaktionsansatz 5 $\mu$l 10x *Taq*-Puffer (100 mM Tris/HCl pH 9,0, 500 mM KCl, 1 % v/v Triton X-100), 3 $\mu$l 25 mM MgCl$_2$, 4 $\mu$l dNTP-Mix (2,5 mM dATP, dCTP, dGTP, dTTP). Nach Auffüllen mit Wasser wurde der Ansatz mit Mineralöl überschichtet und in einem programmierbaren Thermostatisierblock für 2 min auf 94°C erhitzt. Anschließend wurden 2,5 u *Taq* DNA-Polymerase (5 u/$\mu$l, Promega) zugegeben und 20 Temperaturzyklen von 1 min bei 94 °C, 1 min bei 60 °C, 1,5 min bei 72 °C, gefolgt von einer Inkubation für 5 min bei 60 °C, durchgeführt. Die gewünschten Amplifizierungsprodukte wurden durch präparative Agarose-Gelelektrophorese unter Verwendung des Jetsorb DNA Extraction Kits (Genomed) nach den Angaben des Herstellers aus Low Melting Point Agarose (Gibco BRL) isoliert.

Der darauffolgende Amplifizierungsschritt wurde in einem 100 $\mu$l-Ansatz durchgeführt, wobei jeweils ca. 6 ng dieser beiden Fragmente als Matrize, je 50 pmol der beiden Primer SEQ ID NO:6 und SEQ ID NO:7 sowie 1 pmol des Oligodesoxynukleotids SEQ ID NO:5 eingesetzt wurden. Die restlichen Komponenten des PCR-Ansatzes wurden wie in den vorangegangenen Amplifizierungsschritten mit der doppelten Menge zugesetzt. Die PCR fand bei 20 Temperaturzyklen von 1 min bei 94 °C, 1 min bei 55 °C, 1,5 min bei 72 °C statt, gefolgt von einer abschließenden Inkubation für 5 min bei 60 °C. Das erwartete Fragment wurde erneut durch präparative Agarose-Gelelektrophorese isoliert.

Zur Klonierung dieses Fragments, welches die Bibliothek der Lipocalinmuteine in Form der Nukleinsäure repräsentierte, wurde es zunächst mit dem Restriktionsenzym *Bst*XI (New England Biolabs) nach den Angaben des Herstellers geschnitten. Die Reinigung des erhaltenen Nukleinsäurefragments (335 Basenpaare, bp) erfolgte wiederum mittels präparativer Agarose-Gelelektrophorese. Analog wurde die DNA des Vektors pBBP20 mit *Bst*XI geschnitten und das größere der beiden Fragmente (3971 bp) isoliert.

Zur Ligierung wurden 0,93 $\mu$g (4,2 pmol) des PCR-Fragments und 11 $\mu$g (4,2 pmol) des Vektorfragments in Gegenwart von 102 Weiss Units T4 DNA-Ligase (New England Biolabs) in einem Gesamtvolumen von 500 $\mu$l (50 mM Tris/HCl pH 7,8, 10 mM MgCl$_2$, 10 mM DTT, 1 mM ATP, 50 $\mu$g/ml BSA) für zwei Tage bei 16 °C inkubiert. Anschließend wurde die DNA gefällt, indem jeweils 24 $\mu$l des Ligierungsansatzes mit 10 $\mu$g tRNA aus Hefe (Boehringer Mannheim), 25 $\mu$l 5 M Ammoniumacetat und 100 $\mu$l Ethanol versetzt wurden. Nach Inkubation bei -20 °C für drei Tage wurde zentrifugiert (25 min, 16000 g, 4 °C). Das Präzipitat wurde mit jeweils 200 $\mu$l Ethanol (70 % v/v, -20 °C) gewaschen und unter Vakuum getrocknet. Die DNA wurde schließlich in 43,6 $\mu$l TE/10 (1 mM Tris/HCl pH 8,0, 0,1 mM EDTA pH 8,0) aufgenommen. Die DNA-Konzentration der erhaltenen Lösung wurde durch analytische Agarose-Gelelektrophorese anhand der Fluoreszenzintensität der mit Ethidiumbromid angefärbten Banden im Vergleich mit einer Probe bekannter Konzentration abgeschätzt.

Die Präparation elektrokompetenter Zellen des *E. coli* K12-Stamms XL1-Blue (Bullock et al., supra) erfolgte gemäß den von Tung und Chow (Trends Genet. 11 (1995), 128-129) und von Hengen (Trends Biochem. Sci. 21 (1996), 75-76) beschriebenen Methoden. 1 l LB-Medium wurde durch Zugabe einer stationären XL1-Blue Übernachtkultur auf eine optische Dichte bei 600 nm, OD600 = 0,08 eingestellt und bei 200 Upm und 26 °C in einem 2 l-Erlenmeyer-Kolben inkubiert. Nach Erreichen von OD600 = 0,6 wurde die Kultur für 30 min auf Eis gekühlt und anschließend für 15 min bei 4000 g und 4 °C zentrifugiert. Das Zellsediment wurde zweimal mit jeweils 500 ml eiskaltem 10 % w/v Glycerin gewaschen und schließlich in 2 ml eiskaltem GYT-Medium (10 % w/v Glycerin, 0,125 % w/v Hefeextrakt, 0,25 % w/v Trypton) resuspendiert.

Zur Elektroporation wurde das Easyjec T Basic System (EquiBio) mit den dazugehörigen Küvetten (Elektrodenabstand 2 mm) verwendet. Alle Arbeitsschritte wurden im Kühlraum bei 4 °C durchgeführt. Jeweils 5 bis 6 $\mu$l der oben genannten DNA-Lösung (245 ng/$\mu$l) wurde mit 40 $\mu$l der Zellsuspension gemischt, 1 min auf Eis inkubiert und anschließend in die Küvette überführt. Nach der Elektroporation wurde die Suspension sofort in 2 ml frischem, eiskaltem SOC-Medium (2 % w/v Trypton, 0,5 % w/v Hefeextrakt, 10 mM NaCl, 10 mM MgSO$_4$, 10 mM MgCl$_2$) verdünnt und für 60 min bei 37 °C und 200 Upm geschüttelt. Die Zellen wurden anschließend jeweils für 2 min bei 3600 g sedimentiert, in 1 ml LB-Medium mit 100 $\mu$g/ml Ampicillin (LB/Amp) resuspendiert und zu je 200 $\mu$l auf Agar-Platten (140 mm Durchmesser) mit LB/Amp-Medium ausplattiert. Unter Einsatz von insgesamt 10,7 $\mu$g der ligierten DNA wurden auf diese Weise mit acht Elektroporationsansätzen $3,73 \cdot 10^8$ Transformanden erhalten, die auf 40 Agar-Platten verteilt waren und gemäß Beispiel 2 weiter verwendet wurden.

Beispiel 2: Phagemidpräsentation und Selektion von Lipocalinmuteinen gegen Fluorescein

Die auf LB/Amp-Agar ausplattierten Zellen, welche mit den Phasmidvektoren transformiert waren, die für die Bibliothek der Lipocalinmuteine als Fusionsproteine kodierten, wurden für 14 h bei 32 °C inkubiert. Dann wurden die Kolonien unter Zusatz von je 10 ml 2xYT/Amp-Medium von den Agar-Platten abgeschabt, in einen sterilen Erlenmeyerkolben überführt und zur vollständigen Resuspendierung für 20 min bei 37 °C, 200 Upm geschüttelt. 500 ml auf 37 °C vorgewärmtes 2xYT/Amp-Medium wurden mit 2,3 ml dieser Suspension inokuliert, so daß die Zelldichte $OD_{550}$ bei 0,08 lag.

Diese Kultur wurde bei 37 °C, 160 Upm bis zu einer Zelldichte von $OD_{550}$ = 0,5 inkubiert, mit VCS-M13 Helferphage (Stratagene) infiziert (Multiplicity of Infection ca. 10) und für weitere 30 min bei 37 °C, 160 Upm geschüttelt. Anschließend wurde Kanamycin (70 $\mu$g/ml) zugegeben, die Inkubatortemperatur auf 26 °C erniedrigt und nach 10 min mit 25 $\mu$g/l Anhydrotetracyclin (250 $\mu$l einer 50 $\mu$g/ml-Stammlösung in Dimethylformamid, DMF) zur Induktion der Genexpression versetzt. Anschließend wurde für weitere 7 h bei 26 °C, 160 Upm inkubiert.

50 ml wurden aus dieser Kultur entnommen und die Zellen durch Zentrifugation (15 min, 12000 g, 4 °C) sedimentiert. Der Überstand, der die Phagemidpartikel enthielt, wurde sterilfiltriert (0,45 $\mu$m), mit 1/4 Volumen (12,5 ml) 20 % w/v PEG 8000, 15 % w/v NaCl versetzt und über Nacht bei 4 °C inkubiert. Nach Zentrifugation (20 min, 18000 g, 4 °C) wurden die präzipitierten Phagemidpartikel in 2 ml kaltem PBS (4 mM $KH_2PO_4$, 16 mM $Na_2HPO_4$, 115 mM NaCl, pH 7,4) gelöst. Die Lösung wurde für 30 min auf Eis inkubiert und auf zwei 1,5 ml-Reaktionsgefäße verteilt. Nach Abzentrifugieren ungelöster Bestandteile (5 min, 18500 g, 4 °C) wurde der Überstand jeweils in ein neues Reaktionsgefäß überführt.

Zur erneuten Fällung der Phagemidpartikel wurde mit 1/4 Volumen 20 % w/v PEG 8000, 15 % w/v NaCl gemischt und für 30 bis 60 min auf Eis inkubiert. Nach Zentrifugation (20 min, 18500 g, 4 °C) wurde der Überstand entfernt und die präzipitierten Phagemidpartikel in insgesamt 1 ml PBS gelöst. Nach Inkubation für 30 min auf Eis wurde die Lösung zentrifugiert (5 min, 18500 g, 4 °C) und der Überstand mit den Phagemidpartikeln direkt für die Affinitätsanreicherung eingesetzt.

Zur Affinitätsanreicherung der die Lipocalimutein Fusionsproteine tragenden rekombinanten Phagemide wurden Immuno-Sticks (NUNC) verwendet. Diese wurden über Nacht mit 800 $\mu$l eines Konjugats aus Rinderserum-Albumin (BSA) und 4-Glutarylamido-fluorescein (100 $\mu$g/ml) in PBS beschichtet.

Zur Herstellung des Konjugats wurde 4-Amino-fluorescein (Fluoresceinamin Isomer I, Fluka) zunächst mit einem fünfzehnfachen molaren Überschuß an Glutarsäureanhydrid bei pH 7,0 gemäß der Arbeitsvorschrift von Ogano et al. (Carbohydrate Res. 105 (1982), 69-85) umgesetzt, um später die sterische Zugänglichkeit der Fluoresceingruppe zu gewährleisten. Das Reaktionsprodukt 4-Glutarylamido-fluorescein, welches eine zur Kopplung mit dem BSA geeignete Carbonsäuregruppe an einer aliphatischen Seitenkette trug, wurde anschließend durch Umkristallisieren aus Aceton/ Wasser gereinigt. Eine Lösung von 17,3 mg (37,5 $\mu$mol) dieser Substanz in 25 $\mu$l DMF wurde dann zur Aktivierung mit 4,31 mg (37,5 $\mu$mol) N-Hydroxysuccinimid in 25 $\mu$l DMF sowie mit 7,2 mg (37,5 $\mu$mol) 1-Ethyl-3-(3-dimethylaminopropyl) carbodiimid versetzt. Der Ansatz wurde für 1 h bei Raumtemperatur (RT) inkubiert. 20 $\mu$l dieser Lösung wurden mit einer Lösung von 10 mg BSA in 980 $\mu$l 5 % w/v $NaHCO_3$ pH 8,1 versetzt und für 3 h bei RT inkubiert. Nach Abtrennung der überschüssigen Reaktanden von dem BSA-Konjugat mittels einer PD-10 Gelfiltrationssäule (Pharmacia) wurde eine Beladung von 8 Molekülen 4-Glutarylamido-fluorescein pro BSA-Molekül anhand der Absorption der Fluoresceingruppe bei 495 nm ($\varepsilon$ = 72000 $M^{-1}$ $cm^{-1}$) bestimmt.

Unbelegte Bindungsstellen auf der Oberfläche des Immuno-Sticks wurden durch Inkubation mit 1,2 ml 2 % w/v BSA in PBST (PBS mit 0,1 % v/v Tween 20) für 2 h bei RT abgesättigt. Nach dreimaligem kurzen Waschen mit jeweils 1,2 ml PBST wurde der Immuno-Stick in einer Mischung aus 250 $\mu$l der Phagemidlösung und 500 $\mu$l Blockierungspuffer (2 % w/v BSA in PBST) für 1 h bei RT inkubiert.

Zur Entfernung nicht gebundener Phagemide wurde achtmal (bei der ersten Selektion) bzw. zehnmal (bei den Selektionszyklen 2 bis 6) mit jeweils 950 $\mu$l PBST für 2 min gewaschen. Adsorbierte Phagemide wurden schließlich durch 10minütige Behandlung des Immuno-Sticks mit 950 $\mu$l 0,1 M Glycin/HCl pH 2,2 eluiert, wobei der pH der Elutionsfraktion sofort anschließend durch Mischen mit 160 $\mu$l 0,5 M Tris neutralisiert wurde.

Zur Amplifizierung wurde diese Phagemidlösung (1,1 ml, je nach Selektionszyklus zwischen $10^6$ und $10^8$ Colony-forming Units) kurz auf 37 °C erwärmt, mit 4 ml einer exponentiell wachsenden Kultur von *E. coli* XL1-Blue ($OD_{550}$ = 0,5) gemischt und für 30 min bei 37 °C, 200 Upm inkubiert. Die mit den Phagemiden infizierten Zellen wurden anschließend sedimentiert (2 min, 4420 g, 4 °C), in 800 $\mu$l des Kulturmediums resuspendiert und auf vier Agar-Platten mit LB/Amp-Medium (140 mm Durchmesser) ausplattiert.

Zur wiederholten Produktion und Affinitätsanreicherung von Phagemidpartikeln wurde verfahren, wie zu Beginn dieses Beispiels beschrieben. In diesen Fällen wurde mit 0,2 bis 1 ml der Suspension der auf den Agar-Platten gewachsenen Zellen jeweils 50 ml 2xYT/Amp-Medium angeimpft. Auf diese Weise wurden fünf weitere Selektionszyklen mit dem BSA-Fluoresceinkonjugat durchgeführt.

Beispiel 3: Produktion derLipocalinmuteine

Zur präparativen Produktion der Lipocalinmuteine wurde die Genkassette zwischen den beiden *Bst*XI-Schnittstellen aus dem pBBP20-Vektor in das Expressionsplasmid pBBP21 subkloniert. Als Kontrolle wurde das auf pBBP21 ursprünglich kodierte Bbp ebenfalls produziert.

Zur Subklonierung wurde aus der Mischung der *E.coli*-Zellen aus Beispiel 2, die mit den Phagemiden des letzten Selektionszyklus infiziert waren, die Phasmid-DNA unter Verwendung des QIAprep Spin Miniprep Kits (QIAGEN) isoliert. Diese wurde mit dem Restriktionsenzym *Bst*XI geschnitten und das kleinere der beiden Fragmente (335 bp) durch präparative Agarose-Gelelektrophorese wie in Beispiel 1 beschrieben gereinigt. In gleicher Weise wurde die DNA des Vektors pBBP21 mit *Bst*XI geschnitten und das größere der beiden Fragmente (4132 bp) isoliert.

Zur Ligierung wurden jeweils 50 fmol der beiden DNA-Fragmente in einem Gesamtvolumen von 20 $\mu$l (30 mM Tris/HCl pH 7,8, 10 mM MgCl$_2$, 10 mM DTT, 1 mM ATP) mit 1,5 Weiss Units T4 DNA Ligase (Promega) versetzt und 5 h bei 16 °C inkubiert. Mit 5 $\mu$l dieses Ligierungsansatzes wurde dann *E.coli*-TG1-F$^-$ (*E. coli* K12 TG1, der durch wiederholte Kultivierung unter nicht selektiven Bedingungen sein Episom verloren hatte) nach der CaCl$_2$-Methode transformiert (Sambrook et al., supra).

Aus zehn der erhaltenen Kolonien wurde die Plasmid-DNA isoliert und die Ligierung durch Restriktionsanalyse mit den Enzymen *Hind*III und *Kpn*I kontrolliert. Alle zehn Plasmide zeigten die erwarteten Fragmentgrößen von 342 und 4125 bp.

Die Sequenzanalyse der Bbp-Genkassetten erfolgte mit Hilfe des T7 Sequencing Kits (Pharmacia) nach Herstellerangaben unter Verwendung der Oligodesoxynukleotide SEQ ID NO:8 und SEQ ID NO:9. Dabei wurden unter den zehn isolierten Plasmiden nur vier verschiedene Sequenzen gefunden, deren Genprodukte als FluA, FluB, FluC und FluD bezeichnet wurden. Die DNA-Sequenz von FluA war zweimal, von FluB viermal, von FluC dreimal und von FluD einmal vertreten. Die Nukleotidsequenzen von FluA, FluB und FluC wurden in Aminosäuresequenzen übersetzt, und die vom Bbp abweichenden Aminosäuren sind in Tabelle 1 wiedergegeben.

Zur Untersuchung der Bindungsaktivität der Lipocalinmuteine in einem ELISA (Beispiel 4) wurde die Proteinproduktion der entsprechenden Klone im 50 ml-Maßstab durchgeführt. Dazu wurden jeweils 4 ml LB/Amp-Medium mit einer Einzelkolonie des TG1-F$^-$-Transformanden, der das jeweilige Plasmid trug, angeimpft und über Nacht bei 30 °C, 200 Upm inkubiert. 500 $\mu$l dieser Vorkultur wurden dann jeweils auf 50 ml LB/Amp-Medium überimpft und bei 22 °C, 200 Upm bis zu einer OD$_{550}$ = 0,5 geschüttelt. Anschließend wurde mit 200 $\mu$g/l Anhydrotetracyclin (50 $\mu$l einer 200 $\mu$g/ml-Stammlösung in DMF) induziert und weitere 3 h bei 22 °C, 200 Upm geschüttelt. Die Zellen wurden durch Zentrifugation (15 min, 4420 g, 4 °C) sedimentiert und jeweils in 1 ml kaltem Periplasma-Aufschlußpuffer (100 mM Tris/HCl pH 8,0, 500 mM Saccharose, 1 mM EDTA) resuspendiert. Nach Zugabe von 25 $\mu$l einer Lösung von 1 mg/ml Lysozym in dem Periplasma-Aufschlußpuffer wurde für 30 min auf Eis inkubiert. Die Sphäroplasten wurden durch Zentrifugation (15 min, 18500 g, 4 °C) sedimentiert, und der Überstand wurde als periplasmatischer Proteinextrakt in ein neues Reaktionsgefäß überführt.

Zur Proteinproduktion im größeren Maßstab wurde eine 50 ml-Vorkultur (LB/Amp-Medium) direkt mit einer Einzelkolonie des mit dem entsprechenden Plasmid transformierten TG1-F$^-$-Stamms angeimpft und bei 30 °C, 200 Upm über Nacht geschüttelt. Im Fall der Anticaline FluA und FluB wurde der *E. coli*-Stamm JM83 (Yanisch-Perron et al., Gene 33 (1985), 103-119), der kein *sup*E-Gen trägt, verwendet. Die gesamte Vorkultur wurde auf 2 1 LB/Amp-Medium in einem 5 1-Erlenmeyerkolben überimpft, woraufhin die Kultur bei 22 °C, 200 Upm inkubiert wurde. Bei einer Zelldichte von OD$_{550}$ = 0,5 wurde mit 200 $\mu$g/l Anhydrotetracyclin (200 $\mu$l einer 2 mg/ml-Stammlösung in DMF) induziert und für weitere 3 h bei 22 °C, 200 Upm geschüttelt.

Die Zellen wurden abzentrifugiert (15 min, 4420 g, 4 °C) und nach Entfernung des Überstands unter Kühlung auf Eis in 20 ml des Periplasma-Aufschlußpuffers resuspendiert. Nach Zugabe von 50 $\mu$g/ml Lysozym (100 $\mu$l einer Lösung von 10 mg/ml Lysozym in dem Periplasma-Aufschlußpuffer) wurde für 30 min auf Eis inkubiert. Anschließend wurden die Sphäroplasten in zwei aufeinanderfolgenden Zentrifugationsschritten abgetrennt (15 min, 4420 g, 4 °C und 15 min, 30000 g, 4 °C). Der so gewonnene periplasmatische Proteinextrakt wurde gegen CP-Puffer (100 mM Tris/HCl pH 8,0, 150 mM NaCl, 1 mM EDTA) dialysiert, sterilfiltriert und zur chromatographischen Reinigung eingesetzt.

Die Reinigung erfolgte mittels des an den C-Terminus der Lipocalinmuteine fusionierten Strep-Tag II-Affinitätsanhängsels (Schmidt et al., supra). Im vorliegenden Fall wurde das Streptavidinmutein "1" eingesetzt (Deutsche Patentanmeldung 196 41 876.3; Voss und Skerra, Protein Eng. 10 (1997), 975-982), welches an eine aktivierte Sepharose (5 mg/ml immobilisiertes Streptavidin, bezogen auf das Bettvolumen der Matrix) gekoppelt war.

Eine mit diesem Material befüllte Chromatographiesäule mit 2 ml Bettvolumen wurde bei 4 °C und einer Flußrate von 20 ml/h mit 10 ml CP-Puffer äquilibriert. Die Chromatographie wurde durch Messung der Absorption bei 280 nm des Eluats in einem Durchfluß-Photometer verfolgt. Nach dem Auftragen des periplasmatischen Proteinextrakts wurde bis zum Erreichen der Basislinie mit CP-Puffer gewaschen und das gebundene Lipocalinmutein anschließend mit 10 ml einer Lösung von 2,5 mM D-Desthiobiotin (Sigma) in CP-Puffer eluiert. Die Fraktionen, die das gereinigte Lipocalinmutein enthielten, wurden mittels der SDS-Polyacrylamid-Gelelektrophorese (Fling und Gregerson, Anal. Biochem. 155 (1986), 83-88) überprüft und vereinigt. Die Proteinausbeuten lagen zwischen 200 $\mu$g und 3 mg je 2 1 Kultur.

Tabelle 1: Sequenzcharakteristika selektierter Lipocalinmuteine

| Aminosäure-Position | Bbp | FluA | FluB | FluC | HepCl | HepC4 |
|---|---|---|---|---|---|---|
| 34 | Asn | Ser | Gln | Ser | Lys | Gln[a] |
| 35 | Ser | Pro | His | Lys | Thr | Ala |
| 36 | Val | Asn | Trp | Asn | Lys | Pro |
| 37 | Glu | Gly | Asp | Gly | Gln[a] | Gly |
| 58 | Asn | Arg | Arg | Arg | Leu | Pro |
| 60 | His | Asp | Arg | Thr | His | Asn |
| 69 | Ile | Met | His | Gln[a] | Phe | Ala |
| 88 | Leu | Arg | Val | Arg | Val | Trp |
| 90 | Tyr | Val | Arg | Val | Ala | Gly |
| 93 | Val | Tyr | Arg | Lys | Phe | Leu |
| 95 | Lys | Arg | Arg | Arg | Ser | Ala |
| 97 | Asn | Thr | Gly | Gly | Gln | Trp |
| 114 | Tyr | Ser | Arg | Arg | Ala | Pro |
| 116 | Lys | Arg | Arg | Arg | Tyr | Arg |
| 125 | Gln | Trp | Trp | Leu | Val | Leu |
| 127 | Phe | His | His | His | Phe | Pro |
| 40[b] | Gly | | | Arg | Glu | |
| 68[b] | Phe | | | Val | | |
| 70[b] | Glu | | Lys | | | |
| 96[b] | Glu | Lys | | | | |
| 100[b] | Asn | | | | Ser | |

[a]Diese Glutaminsäurereste wurden von Amber-Stopcodons kodiert.
[b]Diese Aminosäuresubstitutionen traten aufgrund zufälliger Mutationen auf.

## Beispiel 4: Ermittlung der Affinität der Lipocalinmuteine für Fluorescein und dessen Derivate

Für den Bindungsnachweis im ELISA (Enzyme-linked Immunosorbent Assay) wurden zunächst die Vertiefungen einer Mikrotiterplatte (Micro Test III Flexible Assay Plate; Falcon) mit je 100 $\mu$l einer 100 $\mu$g/ml-Lösung des BSA-Fluorescein-Konjugats aus Beispiel 2 in PBS gefüllt und über Nacht bei RT inkubiert. Als Kontrolle diente nicht konjugiertes BSA. Die Lösung wurde entfernt und unbelegte Bindungsstellen wurden mit 200 $\mu$l 2 % w/v BSA in PBST für 2 h abgesättigt. Nach dreimaligem Waschen mit PBST wurden 100 $\mu$l des periplasmatischen Proteinextrakts aus der Produktion im 50 ml-Maßstab (Beispiel 3) in die Vertiefungen gefüllt. Ausgehend von diesen Proteinlösungen wurden Verdünnungsreihen in PBST hergestellt. Nach 1 h Inkubation bei RT wurde erneut dreimal mit PBST gewaschen und ein Streptavidin-Alkalische Phosphatase-Konjugat (Amersham), 1:1000 mit PBST verdünnt, in die Vertiefungen gefüllt. Dieses Enzymkonjugat diente zur Erkennung des Strep-Tag II-Anhängsels am C-Terminus der Lipocalinmuteine. Es wurde für 1 h bei RT inkubiert und anschließend zweimal mit PBST und zweimal mit PBS gewaschen. Der Nachweis der an die Fluoresceingruppen gebundenen Lipocalinmuteine erfolgte schließlich mittels der durch die Alkalische Phosphatase katalysierten Hydrolyse von p-Nitrophenylphosphat. Dazu wurden 100 $\mu$l einer Lösung von 0,5 mg/ml p-Nitrophenylphosphat (Amresco) in AP-Puffer (100 mM NaCl, 5 mM $MgCl_2$, 100 mM Tris/HCl pH 8,8) in die Vertiefungen gefüllt und die Produktbildung durch Messung der Absorption bei 405 nm in einem SpectraMax 250-Photometer (Molecular Devices) verfolgt.

Hierbei ließ sich praktisch keine Bindung für FluD und das Bbp nachweisen, während FluA, FluB und FluC intensive Bindungssignale zeigten. Das Signal war in der Relation für FluC am stärksten, gefolgt von FluA und FluB.

Die Liganden-Bindungseigenschaften der Lipocalinmuteine wurden daraufhin mittels der Methode der Fluoreszenztitration bestimmt. Gemessen wurde dabei die Abnahme der intrinsischen Tyrosin- und Tryptophan-Fluoreszenz des Proteins bei Komplexbildung mit dem Liganden. Die Messungen erfolgten mit einem Fluoreszenzphotometer (MS III, Photon Technology International Inc.) bei einer Anregungswellenlänge von 280 nm (Spaltbreite 5 nm) und einer Emissionswellenlänge von 340 nm (Spaltbreite 10 nm). Als Liganden wurden Fluorescein, 4-Amino-fluorescein sowie dessen Konjugat mit Glutarsäure aus Beispiel 2 eingesetzt. Diese drei Liganden zeigten bei den angegebenen Wellenlängen keine signifikante Eigenfluoreszenz.

Als Puffersystem diente PBS unter Zusatz von 1 mM EDTA mit pH 7,4 (mit NaOH eingestellt). Alle verwendeten Lösungen wurden sterilfiltriert (0,45 μm). Die Lösung des jeweiligen gereinigten Lipocalinmuteins aus Beispiel 3 wurde dreimal gegen diesen Puffer dialysiert und durch Verdünnen auf eine Konzentration von 1 μM eingestellt. Die Konzentrationsbestimmung erfolgte mittels der Absorption bei 280 nm unter Verwendung kalkulatorischer Extinktionskoeffizienten von 63680 M$^{-1}$ cm$^{-1}$ für FluB sowie 52300 M$^{-1}$ cm$^{-1}$ für FluC. Für FluA und Bbp wurden die nach Gill und von Hippel (Anal. Biochem. 182 (1989), 319-326) in Gegenwart von Guanidiniumchlorid korrigierten kalkulatorischen Extinktionskoeffizienten von 59755 M$^{-1}$ cm$^{-1}$ (FluA) sowie 54150 M$^{-1}$ cm$^{-1}$ (Bbp) verwendet.

Zur Messung wurde 2 ml der Lipocalinmuteinlösung in einer Quarzküvette, die mit einem Rührfisch ausgestattet war, vorgelegt und im Probenhalter des Photometers auf 25 °C temperiert. Anschließend wurden insgesamt 40 μl einer 250 μM bis 1 mM Lösung des Liganden in demselben Puffer in Schritten von 1 μl bis 4 μl zupipettiert. Die dabei stattfindende Verdünnung der vorgelegten Proteinlösung um maximal 2 % blieb bei der nachfolgenden Auswertung der Daten unberücksichtigt. Nach jedem Titrationsschritt wurde zur Gleichgewichtseinstellung für 1 min unter Rühren inkubiert und das Fluoreszenzsignal als Mittelwert über 10 s gemessen. Nach Abzug des Fluoreszenzwertes für den Puffer wurden die Signale auf einen Anfangswert von 100 % normiert und um den inneren Filtereffekt der Liganden korrigiert. Dazu wurden Fluoreszenztitrationen mit dem jeweiligen Ligand durchgeführt, bei denen die Lipocalinmutein -Lösung durch N-Acetyl-L-tryptophanamid (Sigma) ersetzt war.

Die so erhaltenen Meßwerte einer Titrationsreihe wurden gemäß folgender Formel durch nicht-lineare Regression mit Hilfe des Computerprogramms Kaleidagraph (Abelbeck Software) angepaßt:

$$F = \left([P]_t - [L]_t - K_d\right)\frac{f_P}{2} + \left([P]_t + [L]_t + K_d\right)\frac{f_{PL}}{2} + (f_P - f_{PL})\sqrt{\frac{\left([P]_t + [L]_t + K_d\right)^2}{4} - [P]_t[L]_t}$$

Dabei bedeuten F die normierte Fluoreszenzintensität und $[P]_t$ die Konzentration des Lipocalinmuteins. $[L]_t$ ist die Gesamtkonzentration des Liganden bei dem jeweiligen Titrationsschritt. $f_{PL}$ und $K_d$ wurden als freie Parameter an die gemessenen Daten angepaßt und stehen für den Fluoreszenzkoeffizienten des Lipocalinmutein-Ligandkomplexes sowie für die thermodynamische Dissoziationskonstante dieses Komplexes. Im Fall von FluC wurde zusätzlich $[P]_t$ als freier Parameter angepaßt. Die ermittelten Dissoziationskonstanten für die Lipocalinmuteine FluA, FluB und FluC sind in Tabelle 2 wiedergegeben. Der Bindungseffekt bei der Vergleichsmessung mit Bbp war so schwach, daß eine Dissoziationskonstante in diesem Fall nicht bestimmt werden konnte.

Tabelle 2: Dissoziationskonstanten für die Komplexe aus Lipocalinmutein und Fluoresceinderivaten

|  | Fluorescein | 4-Aminofluorescein | 4-Glutarylamidofluorescein |
|---|---|---|---|
| FluA | 118 ± 14 nM | 224 ± 6 nM | 601 ± 16 nM |
| FluB | 5,73 ± 0,86 μM | 2,84 ± 0,3 μM | 4,70 ± 0,51 μM |
| FluC | 411 ± 20 μM | 299 ± 41 nM | 78 ± 3 nM |

Beispiel 5: Selektion von Lipocalinmutein gegen ein Hepatitis C-Peptidepitop

Zur Selektion der Lipocalinmuteine wurde die in Beispiel 1 hergestellte Bibliothek verwendet. Die Vermehrung und Isolierung der Phagemide erfolgte genauso wie in Beispiel 2 beschrieben.

Als Peptidligand wurde ein biotinyliertes synthetisches Hepatitis C-Peptidepitop eingesetzt, bei dem es sich um das Peptidfragment Nr. 59 aus dem Oberflächenprotein NS4 von HCV handelte (Khudyakow et al., Virology 206 (1995), 666-672). Das Peptid, SEQ ID NO:13, wurde nach der üblichen Fmoc-Methode mittels eines PS3 Automaten (RAININ Instrument Co.) synthetisiert, wobei Rink Amid MBHA-Harz (novabiochem) eingesetzt wurde. Im Anschluß an die Kopplung der Aminosäurebausteine vom C- zum N-Terminus wurde Aminocapronsäure als Boc-geschütztes Derivat und im letzten Schritt D-Biotin (Sigma) gekoppelt. Das vom Harz abgespaltene und entschützte Peptid wurde mittels HPLC gereinigt, und seine Zusammensetzung wurde durch ESI-Massenspektrometrie überprüft.

Zur Affinitätsanreicherung der die Lipocalinmutein-Fusionsproteine tragenden rekombinanten Phagemide wurden mit Streptavidin beschichtete superparamagnetische Partikel (Dynabeads M-280 Streptavidin, Dynal) verwendet. Die Menge des Peptidliganden wurde so eingestellt, daß dieser einerseits im molaren Überschuß gegenüber den eingesetzten Phagemiden vorlag, und daß andererseits die Bindekapazität des Streptavidins für die Biotingruppen nicht überschritten wurde.

Dazu wurden 20 μl der Peptidlösung (20 μg/ml in PBS) mit 280 μl einer Lösung der frisch präparierten Phagemide (3,0·10¹² cfu/ml) gemischt und für 1 h bei RT inkubiert, woraufhin 100 $\mu$l einer Lösung von 8 % w/v BSA, 0,4 % v/v Tween 20 in PBS zugegeben wurde. Parallel wurden 100 $\mu$l der kommerziell erhältlichen Suspension der magnetischen Partikel dreimal mit jeweils 100 $\mu$l PBS gewaschen und zur Absättigung unspezifischer Bindungsstellen mit 100 $\mu$l 2 % w/v BSA in PBST für 1 h bei RT inkubiert. Nach Entfernung des Überstandes wurden die magnetischen Partikel mit der Peptid/Phagemidmischung versetzt, resuspendiert und für 10 min bei RT inkubiert. Zur Absättigung freier Biotin-Bindungsstellen des Streptavidins wurde die Mischung schließlich mit 10 $\mu$l einer Lösung von 4 $\mu$M Desthiobiotin in PBS versetzt und für 5 min bei RT inkubiert.

Zur Entfernung nicht gebundener Phagemide wurden die magnetischen Partikel achtmal mit jeweils 1 ml PBST, 0,1 $\mu$M Desthiobiotin gewaschen. Dazu wurden die magnetischen Partikel mit Hilfe eines Magneten an der Wand des 1,5 ml Eppendorfgefäßes gesammelt und der Überstand abgezogen. Danach wurden die magnetischen Partikel mit frischem Puffer resuspendiert und für 1 min durch Rotation des Gefäßes in Suspension gehalten. Die Elution der gebundenen Phagemide erfolgte durch 10minütige Inkubation der resuspendierten Partikel in 950 $\mu$l 0,1 M Glycin/HCl pH 2,2. Der pH-Wert der Lösung wurde im Anschluß daran sofort durch Zugabe von 160 $\mu$l 0,5 M Tris neutralisiert.

Anschließend wurden die eluierten Phagemide wie in Beispiel 2 beschrieben vermehrt und für eine erneute Affinitätsselektion unter den oben angegebenen Bedingungen eingesetzt. Insgesamt wurden 6 Selektionszyklen durchgeführt.

Beispiel 6: Identifizierung peptidbindender Lipocalinmuteine mittels der "Colony Screening"-Methode

Zur analytischen Produktion der Lipocalinmuteine als Fusionsprotein mit dem Strep-Tag II sowie der Albumin-Bindungsdomäne und deren Charakterisierung durch "Colony Screening" wurde die Genkassette zwischen den beiden *Bst*XI-Schnittstellen aus dem Vektor pBBP20 in pBBP22 subkloniert.

Dazu wurde aus der Mischung der *E.coli*-Klone, die durch Infektion mit den im letzten Selektionszyklus eluierten Phagemiden aus Beispiel 6 erhalten worden waren, die Phasmid-DNA unter Verwendung des QIAprep Spin Miniprep Kits (QIAGEN) isoliert. Die DNA wurde mit dem Restriktionsenzym *Bst*XI geschnitten und das kleinere der beiden Fragmente (335 bp) durch präparative Agarose-Gelelektrophorese wie in Beispiel 1 beschrieben gereinigt. In gleicher Weise wurde die DNA des Vektors pBBP22 mit *Bst*XI geschnitten und das größere der beiden Fragmente (3545 bp) isoliert.

Zur Ligierung wurden jeweils 50 fmol der beiden DNA-Fragmente in einem Gesamtvolumen von 20 $\mu$l (30 mM Tris/HCl pH 7,8, 10 mM MgCl₂, 10 mM DTT, 1 mM ATP) mit 1,5 Weiss Units T4 DNA Ligase (Promega) versetzt und über Nacht bei 16 °C inkubiert. Mit 5 $\mu$l dieses Ligierungsansatzes wurde *E.coli* TG1-F⁻ nach der CaCl₂-Methode transformiert.

Auf eine LB/Amp-Agarplatte wurde eine passend zurechtgeschnittene, an einer Stelle markierte hydrophile PVDF-Membran (Millipore, Typ GVWP, Porengröße 0,22 $\mu$m) aufgelegt und auf dieser Membran 150 $\mu$l der Zellsuspension aus dem Transformationsansatz gleichmäßig ausplattiert. Die Menge des ausplattierten Transformationsansatzes war so bemessen, daß ca. 500 Kolonien erhalten wurden. Die Platte wurde für 6,5 h bei 37 °C im Brutschrank inkubiert, bis die Kolonien eine mit dem Auge gut erkennbare Größe erreicht hatten.

In der Zwischenzeit wurde eine ebenfalls passend zurechtgeschnittene hydrophobe Membran (Millipore, Immobilon P, Porengröße 0,45 $\mu$m) nach den Angaben des Herstellers mit PBS angefeuchtet. Anschließend wurde sie für 4 h bei RT in einer Lösung von 10 mg/ml Human-Serumalbumin (HSA, Sigma) in PBS geschwenkt. Verbliebene Bindungsstellen auf der Membran wurden durch Inkubation mit 3 % w/v BSA, 0,5 % v/v Tween 20 in PBS für 2 h bei RT abgesättigt. Die Membran wurde zweimal für jeweils 10 min mit 20 ml PBS gewaschen und danach für 10 min in 10 ml LB/Amp-Medium, dem 200 $\mu$g/l Anhydrotetracyclin zugesetzt war, geschwenkt. Anschließend wurde sie an einer Stelle markiert und auf eine Kulturplatte mit LB/Amp-Agar, der zusätzlich 200 $\mu$g/l Anhydrotetracyclin enthielt, gelegt. Die mit den Kolonien bewachsene hydrophile Membran wurde so auf die hydrophobe Membran aufgelegt, daß die beiden Markierungen zur Deckung kamen. Die Kulturplatte mit den beiden Membranen wurde bei 22 °C für 15 h inkubiert. Während dieser Phase wurden die jeweiligen Lipocalinmuteine von den Kolonien sekretiert und mittels der Albumin-Bindungsdomäne an dem HSA auf der unteren Membran immobilisiert.

Danach wurde die obere Membran mit den Kolonien auf eine frische LB/Amp-Agarplatte transferiert und bei 4 °C aufbewahrt. Die hydrophile Membran wurde abgenommen, dreimal für jeweils 10 min mit 20 ml PBST gewaschen und anschließend 1 h in 10 ml einer Lösung von 1 $\mu$M SEQ ID NO:13 in PBST inkubiert. Nach zweimaligem Waschen in PBST wurde für 1 h mit 10 ml Avidin-Alkalische Phosphatase-Konjugat (ExtrAvidin-AP-Konjugat, Sigma, 1:1000 verdünnt in PBST) inkubiert. Die Membran wurde anschließend für jeweils 5 min zweimal mit PBST und zweimal mit PBS gewaschen und für 10 min in AP-Puffer (0,1 M Tris/HCl pH 8,8, 0,1 M NaCl, 5 mM MgCl₂) geschwenkt. Zur chromogenen Nachweisreaktion wurde die Membran in 10 ml AP-Puffer, dem 30 $\mu$l BCIP (50 $\mu$g/ml in Dimethylformamid) und 5 $\mu$l NBT (75 $\mu$g/ml in 70 % v/v Dimethylformamid) zugesetzt waren, inkubiert, bis an den Positionen einiger der Kolonien deutliche Farbsignale zu erkennen waren. Auf diese Weise wurde die Bindungsaktivität der von diesen Kolonien produzierten Lipocalinmuteine für den Peptidliganden nachgewiesen.

Acht dieser Kolonien wurden kultiviert. Ihre Plasmid-DNA wurde isoliert und die Bbp-Genkassette einer Sequenzanalyse wie in Beispiel 3 unterzogen. Alle Klone wiesen dabei unterschiedliche Sequenzen auf. Die charakteristischen Aminosäuren der Lipocalinmuteine HepCl und HepC4 sind in Tabelle 1 angegeben.

Beispiel 7: Verwendung der Lipocalinmuteine zum Nachweis des Hepatitis C-Peptidepitops in einem Sandwich-ELISA

Ausgehend von den in Beispiel 6 gefundenen Klonen wurden die entsprechenden Lipocalinmuteine als Fusionsproteine mit dem Strep-Tag II und der Albumin-Bindungsdomäne produziert. Die Genexpression erfolgte im 50 ml-Maßstab. Dazu wurden jeweils 4 ml LB/Amp-Medium mit einer Einzelkolonie von TG1-F⁻, die das jeweilige Plasmid trug, angeimpft und über Nacht bei 30 °C, 200 Upm inkubiert. 500 $\mu$l dieser Vorkultur wurden dann jeweils auf 50 ml LB/Amp-Medium überimpft und bei 22 °C, 200 Upm bis zu einer $OD_{550}$ = 0,5 geschüttelt. Anschließend wurde mit 200 $\mu$g/l Anhydrotetracyclin (50 $\mu$l einer 200 $\mu$g/ml-Stammlösung in DMF) induziert und weitere 3 h bei 22 °C, 200 Upm geschüttelt. Die Zellen wurden durch Zentrifugation (15 min, 4420 g, 4 °C) sedimentiert und jeweils in 1 ml kaltem Periplasma-Aufschlußpuffer (100 mM Tris/HCl pH 8,0, 500 mM Saccharose, 1 mM EDTA) resuspendiert. Nach Zugabe von 25 $\mu$l einer Lösung von 1 mg/ml Lysozym in dem Periplasma-Aufschlußpuffer wurde für 30 min auf Eis inkubiert. Die Sphäroplasten wurden durch Zentrifugation (15 min, 18500 g, 4 °C) sedimentiert, und der Überstand wurde als periplasmatischer Proteinextrakt in ein neues Reaktionsgefäß überführt.

Für den ELISA wurden die Vertiefungen einer Mikrotiterplatte (ELISA-STRIP, 2x8 Well, KO, F-Form, Bindekapazität hoch, Greiner) mit jeweils 200 $\mu$l einer Lösung von 20 mg/ml HSA in 50 mM $NaHCO_3$ pH 9,6 gefüllt und für 1 h bei RT inkubiert. Nach Entfernen der Lösung wurden unbelegte Bindungsstellen mit 200 $\mu$l 3 % w/v BSA in PBS mit 0,5 % v/v Tween 20 für 1 h abgesättigt. Nach dreimaligem Waschen mit PBST wurde jeweils in die erste Vertiefung einer Reihe 50 $\mu$l des unverdünnten periplasmatischen Proteinextrakts gefüllt. In den darauffolgenden Vertiefungen jeder Reihe wurde zunächst je 50 $\mu$l PBS vorgelegt. Anschließend wurde jeweils in die zweite Vertiefung 50 $\mu$l des periplasmatischen Proteinextrakts pipettiert, gemischt und davon ausgehend in den weiteren Vertiefungen der Reihe schrittweise 1:2-Verdünnungen zubereitet. Als Kontrolle diente der periplasmatische Proteinextrakt mit dem Bbp, der unter Verwendung von pBBP22 als Expressionsplasmid hergestellt worden war.

Nach 1 h Inkubation bei RT wurde erneut dreimal mit PBST gewaschen und anschließend jeweils 200 $\mu$l der Ligandenlösung (SEQ ID NO:13, 1 $\mu$M in PBST) in die Vertiefung pipettiert. Nach 1 h Inkubation bei RT wurde dreimal mit PBST gewaschen und danach 50 $\mu$l Avidin-Alkalische Phosphatase-Konjugat (ExtrAvidin-AP-Konjugat, Sigma), 1:1000 verdünnt in PBST, in jede Vertiefung gefüllt. Es wurde erneut für 1 h bei RT inkubiert und anschließend zweimal mit PBST und zweimal mit PBS gewaschen. Der Nachweis der gebundenen Lipocalinmuteine erfolgte mittels chromogener Reaktion in Gegenwart von p-Nitrophenylphosphat. Dazu wurden 100 $\mu$l einer Lösung von 0,5 mg/ml p-Nitrophenylphosphat (Amresco) in AP-Puffer in jede Vertiefung gefüllt und die Produktbildung durch Messung der Absorption bei 405 nm in einem SpectraMax 250-Photometer (Molecular Devices) als dA/dt-Wert gemessen.

Im Fall des Bbp ließen sich nur niedrige Signale nachweisen, während alle analysierten Lipocalinmuteine eindeutige Bindung zeigten. Das Signal war für HepC1 am stärksten, gefolgt von HepC4. Die Bindungskurven für HepC1, HepC4 und Bbp sind in Figur 7 dargestellt.

Beispiel 8: Verwendung des Lipocalinmuteins FluA zur Löschung der Eigenfluoreszenz von Fluorescein

Durch Fluoreszenztitration einer Lösung von Fluorescein mit unterschiedlichen Konzentrationen des Lipocalinmuteins FluA wurde die Komplexbildung verfolgt. Gemessen wurde dabei die Abnahme der Intensität der Eigenfluoreszenz des Liganden Fluorescein. Die Messungen erfolgten mit einem LS 50 B Fluoreszenzphotometer (Perkin Elmer) bei einer Anregungswellenlänge von 490 nm (Spaltbreite 4 nm) und einer Emissionswellenlänge von 512 nm (Spaltbreite 4 nm).

Als Puffersystem diente PBS unter Zusatz von 1 mM EDTA mit pH 7,4 (mit NaOH eingestellt). Alle verwendeten Lösungen wurden sterilfiltriert (0,45 $\mu$m). Die Lösung des gereinigten Lipocalinmuteins FluA aus Beispiel 3 wurde dreimal gegen diesen Puffer dialysiert. Die Konzentrationsbestimmung erfolgte unter Verwendung eines Extinktionskoeffizienten von 59755 $M^{-1}$ $cm^{-1}$ für FluA.

Für eine Meßreihe wurde ein Satz von 15 Lösungen mit einer konstanten Fluoresceinkonzentration von 1 $\mu$M und variierenden Proteinkonzentrationen von 0 bis 10 $\mu$M jeweils in einem Gesamtvolumen von 120 $\mu$l hergestellt. Dazu wurden je 6 $\mu$l einer 20 $\mu$M Lösung von Fluorescein in dem genannten Puffer mit unterschiedlichen Volumina der Lipocalinmutein-Stammlösung versetzt und mit Puffer auf ein Gesamtvolumen von 120 $\mu$l aufgefüllt.

Zur Messung der Fluoreszenzintensität in Abhängigkeit von der jeweiligen Konzentration des Lipocalinmuteins wurden die einzelnen Lösungen in eine Quarz-Mikroküvette überführt und im Probenhalter des Photometers für 1 min auf 25 °C temperiert. Anschließend wurde das Fluoreszenzsignal als Mittelwert über 10 s gemessen. Nach Abzug des Fluoreszenzwertes für den Puffer wurden die Signale auf einen Anfangswert von 100 % skaliert. Eine darüber hinaus gehende Korrektur der Meßwerte erwies sich nicht als notwendig.

Es zeigte sich, daß die anfänglich hohe Fluoreszenzintensität des Fluoresceins mit zunehmender Konzentration des Lipocalinmuteins erheblich abnahm, bis nur noch eine sehr geringe Fluoreszenz gemessen werden konnte. Die erhaltenen Meßwerte der Titrationsreihe wurden gemäß folgender Formel durch nicht-lineare Regression mit Hilfe des Computerprogramms Kaleidagraph (Abelbeck Software) angepaßt:

$$F = \left([L]_t - [P]_t - K_d\right)\frac{f_L}{2} + \left([P]_t + [L]_t + K_d\right)\frac{f_{PL}}{2} + \left(f_L - f_{PL}\right)\sqrt{\frac{\left([P]_t + [L]_t + K_d\right)^2}{4} - [P]_t[L]_t}$$

Dabei bedeuten F die skalierte Fluoreszenzintensität und $[L]_t$ die Konzentration des Fluoresceins (1 $\mu$m).$[P]_t$ steht für die Gesamtkonzentration von FluA bei dem jeweiligen Titrationsschritt. $f_{PL}$ und Kd wurden als freie Parameter an die gemessenen Daten angepaßt und bezeichnen den Fluoreszenzkoeffizienten des Komplexes aus dem Lipocalinmutein A und Fluorescein sowie dessen thermodynamische Dissoziationskonstante.

Die gemessenen Fluoreszenz-Intensitätswerte und die daran angepaßte Ausgleichskurve sind in Figur 8 dargestellt. Als Dissoziationskonstante für den Komplex aus dem Lipocalinmutein FluA und Fluorescein wurde ein Wert von 35,2 $\pm$ 3,2 nM ermittelt. Bei der Komplexbildung mit dem Lipocalinmutein FluA wurde die Fluoreszenzintensität von Fluorescein zu 99,7 $\pm$ 0,3 %, d. h. nahezu quantitativ gelöscht. In einem entsprechenden Kontrollexperiment mit dem rekombinanten BBP wurde keine vergleichbare Fluoreszenzlöschung gefunden.

Sequenzprotokoll

(1) ALLGEMEINE ANGABEN:

(i) ANMELDER:

(A) NAME: Prof. Dr. Arne Skerra
(B) STRASSE: Max-Lehner-Str. 18
(C) ORT: Freising
(D) LAND: Deutschland
(E) POSTLEITZAHL: 85354

(ii) BEZEICHNUNG DER ERFINDUNG: Anticaline

(iii) ANZAHL DER SEQUENZEN: 13

(iv) COMPUTERLESBARE FASSUNG:

(A) DATENTRÄGER: Diskette
(B) COMPUTER: IBM PC-kompatibel
(C) BETRIEBSSYSTEM: PC-DOS/MS-DOS
(D) SOFTWARE:

(2) ANGABEN ZU SEQ ID-NO:1:

(i) SEQUENZCHARAKTERISTIKA:

(A) LÄNGE: 64 Basen
(B) ART: Nucleinsäure
(C) STRANGFORM: Einzelstrang
(D) TOPOLOGIE: linear

(ii) ART DES MOLEKÜLS: synthetisches Oligodesoxynukleotid

(xi) SEQUENZBESCHREIBUNG: SEQ ID-NO:1:

```
CCATGGTAAA TGGTGGGAAG TCGCCAAATA CCCCNNKNMS NNSNNKAAGT    50
ACGGAAAGTG CGGA                                          64
```

(2) ANGABEN ZU SEQ ID-NO:2:

    (i) SEQUENZCHARAKTERISTIKA:

        (A) LÄNGE: 71 Basen
        (B) ART: Nucleinsäure
        (C) STRANGFORM: Einzelstrang
        (D) TOPOLOGIE: linear

    (ii) ART DES MOLEKÜLS: synthetisches Oligodesoxynukleotid

    (xi) SEQUENZBESCHREIBUNG: SEQ ID-NO:2:

```
GGGTAGGCGG TACCTTCSNN AAAGTATTCC TTGCCGTGGA TTACMNNGTA    50
SNNCGAAACT TTGACACTCT T                                  71
```

(2) ANGABEN ZU SEQ ID-NO:3:

    (i) SEQUENZCHARAKTERISTIKA:

        (A) LÄNGE: 74 Basen
        (B) ART: Nucleinsäure
        (C) STRANGFORM: Einzelstrang
        (D) TOPOLOGIE: linear

    (ii) ART DES MOLEKÜLS: synthetisches Oligodesoxynukleotid

    (xi) SEQUENZBESCHREIBUNG: SEQ ID-NO:3:

```
CCAAGATTGG AAAGATCTAC CACAGCNNSA CTNNKGGAGG TNNSACCVVS    50
GAGNNKGTAT TCAACGTACT CTCC                               74
```

(2) ANGABEN ZU SEQ ID-NO:4:

    (i) SEQUENZCHARAKTERISTIKA:

        (A) LÄNGE: 78 Basen
        (B) ART: Nucleinsäure
        (C) STRANGFORM: Einzelstrang
        (D) TOPOLOGIE: linear

    (ii) ART DES MOLEKÜLS: synthetisches Oligodesoxynukleotid

    (xi) SEQUENZBESCHREIBUNG: SEQ ID-NO:4:

```
TCTGGAGAGC ACCCAGACMN NGTCSNNGTG TCCCTTCTTG TCCTCGTCGT      50
ASNNGCAMNN GTATCCGATG ATGTAGTT                               78
```

(2) ANGABEN ZU SEQ ID-NO:5:

(i) SEQUENZCHARAKTERISTIKA:

(A) LÄNGE: 46 Basen
(B) ART: Nucleinsäure
(C) STRANGFORM: Einzelstrang
(D) TOPOLOGIE: linear

(ii) ART DES MOLEKÜLS: synthetisches Oligodesoxynukleotid

(xi) SEQUENZBESCHREIBUNG: SEQ ID-NO:5:

```
AGATCTTTCC AATCTTGGAG TCACCAACTG GGTAGGCGGT ACCTTC      46
```

(2) ANGABEN ZU SEQ ID-NO:6:

(i) SEQUENZCHARAKTERISTIKA:

(A) LÄNGE: 36 Basen
(B) ART: Nucleinsäure
(C) STRANGFORM: Einzelstrang
(D) TOPOLOGIE: linear
(E) ART DES MOLEKÜLS: synthetisches Oligodesoxynukleotid

(xi) SEQUENZBESCHREIBUNG: SEQ ID-NO:6:

```
CTTCGACTGG TCCCAGTACC ATGGTAAATG GTGGGA      36
```

(2) ANGABEN ZU SEQ ID-NO:7:

(i) SEQUENZCHARAKTERISTTKA:

(A) LÄNGE: 37 Basen
(B) ART: Nucleinsäure
(C) STRANGFORM: Einzelstrang
(D) TOPOLOGIE: linear

(ii) ART DES MOLEKÜLS: synthetisches Oligodesoxynukleotid

(xi) SEQUENZBESCHREIBUNG: SEQ ID-NO:7:

```
CACCAGTAAG GACCATGCTT CTGGAGAGCA CCCAGAC      37
```

(2) ANGABEN ZU SEQ ID-NO:8:

(i) SEQUENZCHARAKTERISTIKA:

(A) LÄNGE: 17 Basen
(B) ART: Nucleinsäure
(C) STRANGFORM: Einzelstrang
(D) TOPOLOGIE: linear

(ii) ART DES MOLEKÜLS: synthetisches Oligodeaoxynukleotid

(xi) SEQUENZBESCHREIBUNG: SEQ ID-NO:8:

GACGGTGCCT GTCCCGA          17

(2) ANGABEN ZU SEQ ID-NO:9:

(i) SEQUENZCHARARTERISTIKA:

(A) LÄNGE: 17 Basen
(B) ART: Nucleinsäure
(C) STRANGFORM: Einzelstrang
(D) TOPOLOGIE: linear

(ii) ART DES MOLEKÜLS: synthetisches Oligodesoxynukleotid

(xi) SEQUENZBESCHREIBUNG: SEQ ID-NO:9:

GACTACTGGG GAGCCGA          17

(2) ANGABEN ZU SEQ ID-NO:10:

(i) SEQUENZCHARAKTERISTIKA:

(A) LÄNGE: 1219 Basenpaare
(B) ART: Nucleinsäure
(C) STRANGFORM: Doppelstrang
(D) TOPOLOGIE: linear

(ii) ART DES MOLEKÜLS: Fragment des Plasmids pBBP20

(ix) MERKMAL:

NAME/SCHLÜSSEL: Signalpeptid
LAGE: (22..84)

(ix) MERKMAL:

NAME/SCHLÜSSEL: reifes Peptid
LAGE: (85..1209)
SONSTIGE ANGABEN:
/Produkt="Fusionsprotein aus Bilin-Bindungsprotein, Strep-Tag II und Fragment des Phagen-Hüllproteins pIII"
/Codon=(Sequenz:"TAG", Aminosäure :Gln)

(ix) MERKMAL:

NAME/SCHLÜSSEL: kodierende Sequenz
LAGE: (85..606)
SONSTIGE ANGABEN:
/Produkt="matures Bilin-Bindungsprotein"

(ix) MERKMAL:

NAME/SCHLÜSSEL: kodierende Sequenz
LAGE: (607..636)
SONSTIGE ANGABEN:

/Produkt="Strep-Tag II-Affinitätsanhängsel"

(ix) MERKMAL:

NAME/SCHLÜSSEL: kodierende Sequenz
LAGE: (637..639)
SONSTIGE ANGABEN:
/Sonstigesm"Amber-Stopcodon"

(ix) MERKMAL:

NAME/SCHLÜSSEL: kodierende Sequenz
LAGE: (640..1209)
SONSTIGE ANGABEN:
/Produkt="Äminosäuren 217-406 des Hüllproteins pIII"

(xi) SEQUENZBESCHREIBUNG: SEQ ID-NO:10:

```
TCTAGTTAAC GAGGGCAAAA A ATG AAA AAG ACA GCT ATC GCG ATT     45
                        Met Lys Lys Thr Ala Ile Ala Ile
                        -21 -20                 -15

GCA GTG GCA CTG GCT GGT TTC GCT ACC GTA GCG CAG GCC GAC GTG 90
Ala Val Ala Leu Ala Gly Phe Ala Thr Val Ala Gln Ala Asp Val
            -10             -5              -1   1

TAC CAC GAC GGT GCC TGT CCC GAA GTC AAG CCA GTC GAC AAC TTC 135
Tyr His Asp Gly Ala Cys Pro Glu Val Lys Pro Val Asp Asn Phe
            5               10              15

GAC TGG TCC CAG TAC CAT GGT AAA TGG TGG GAA GTC GCC AAA TAC 180
Asp Trp Ser Gln Tyr His Gly Lys Trp Trp Glu Val Ala Lys Tyr
            20              25              30

CCC AAC TCA GTT GAG AAG TAC GGA AAG TGC GGA TGG GCT GAG TAC 225
Pro Asn Ser Val Glu Lys Tyr Gly Lys Cys Gly Trp Ala Glu Tyr
            35              40              45

ACT CCT GAA GGC AAG AGT GTC AAA GTT TCG AAC TAC CAC GTA ATC 270
Thr Pro Glu Gly Lys Ser Val Lys Val Ser Asn Tyr His Val Ile
            50              55              60
```

```
CAC GGC AAG GAA TAC TTT ATT GAA GGA ACT GCC TAC CCA GTT GGT 315
His Gly Lys Glu Tyr Phe Ile Glu Gly Thr Ala Tyr Pro Val Gly
            65              70              75

GAC TCC AAG ATT GGA AAG ATC TAC CAC AGC CTG ACT TAC GGA GGT 360
Asp Ser Lys Ile Gly Lys Ile Tyr His Ser Leu Thr Tyr Gly Gly
            80              85              90

GTC ACC AAG GAG AAC GTA TTC AAC GTA CTC TCC ACT GAC AAC AAG 405
Val Thr Lys Glu Asn Val Phe Asn Val Leu Ser Thr Asp Asn Lys
            95              100             105

AAC TAC ATC ATC GGA TAC TAC TGC AAA TAC GAC GAG GAC AAG AAG 450
Asn Tyr Ile Ile Gly Tyr Tyr Cys Lys Tyr Asp Glu Asp Lys Lys
            110             115             120

GGA CAC CAA GAC TTC GTC TGG GTG CTC TCC AGA AGC ATG GTC CTT 495
Gly His Gln Asp Phe Val Trp Val Leu Ser Arg Ser Met Val Leu
            125             130             135

ACT GGT GAA GCC AAG ACC GCT GTC GAG AAC TAC CTT ATC GGC TCC 540
Thr Gly Glu Ala Lys Thr Ala Val Glu Asn Tyr Leu Ile Gly Ser
            140             145             150

CCA GTA GTC GAC TCC CAG AAA CTG GTA TAC AGT GAC TTC TCT GAA 585
Pro Val Val Asp Ser Gln Lys Leu Val Tyr Ser Asp Phe Ser Glu
            155             160             165

GCC GCC TGC AAG GTC AAC AAT AGC AAC TGG TCT CAC CCG CAG TTC 630
Ala Ala Cys Lys Val Asn Asn Ser Asn Trp Ser His Pro Gln Phe
            170             175             180

GAA AAA TAG GCT GGC GGC GGC TCT GGT GGT GGT TCT GGC GGC GGC 675
Glu Lys Gln Ala Gly Gly Gly Ser Gly Gly Gly Ser Gly Gly Gly
            185             190             195

TCT GAG GGT GGT GGC TCT GAG GGT GGC GGT TCT GAG GGT GGC GGC 720
Ser Glu Gly Gly Gly Ser Glu Gly Gly Gly Ser Glu Gly Gly Gly
            200             205             210

TCT GAG GGA GGC GGT TCC GGT GGT GGC TCT GGT TCC GGT GAT TTT 765
Ser Glu Gly Gly Gly Ser Gly Gly Gly Ser Gly Ser Gly Asp Phe
            215             220             225

GAT TAT GAA AAG ATG GCA AAC GCT AAT AAG GGG GCT ATG ACC GAA 810
Asp Tyr Glu Lys Met Ala Asn Ala Asn Lys Gly Ala Met Thr Glu
            230             235             240

AAT GCC GAT GAA AAC GCG CTA CAG TCT GAC GCT AAA GGC AAA CTT 855
Asn Ala Asp Glu Asn Ala Leu Gln Ser Asp Ala Lys Gly Lys Leu
            245             250             255

GAT TCT GTC GCT ACT GAT TAC GGT GCT GCT ATC GAT GGT TTC ATT 900
Asp Ser Val Ala Thr Asp Tyr Gly Ala Ala Ile Asp Gly Phe Ile
            260             265             270
```

```
GGT GAC GTT TCC GGC CTT GCT AAT GGT AAT GGT GCT ACT GGT GAT 945
Gly Asp Val Ser Gly Leu Ala Asn Gly Asn Gly Ala Thr Gly Asp
        275                 280                 285

TTT GCT GGC TCT AAT TCC CAA ATG GCT CAA GTC GGT GAC GGT GAT 990
Phe Ala Gly Ser Asn Ser Gln Met Ala Gln Val Gly Asp Gly Asp
        290                 295                 300

AAT TCA CCT TTA ATG AAT AAT TTC CGT CAA TAT TTA CCT TCC CTC 1035
Asn Ser Pro Leu Met Asn Asn Phe Arg Gln Tyr Leu Pro Ser Leu
        305                 310                 315

CCT CAA TCG GTT GAA TGT CGC CCT TTT GTC TTT GGC GCT GGT AAA 1080
Pro Gln Ser Val Glu Cys Arg Pro Phe Val Phe Gly Ala Gly Lys
        320                 325                 330

CCA TAT GAA TTT TCT ATT GAT TGT GAC AAA ATA AAC TTA TTC CGT 1125
Pro Tyr Glu Phe Ser Ile Asp Cys Asp Lys Ile Asn Leu Phe Arg
        335                 340                 345

GGT GTC TTT GCG TTT CTT TTA TAT GTT GCC ACC TTT ATG TAT GTA 1170
Gly Val Phe Ala Phe Leu Leu Tyr Val Ala Thr Phe Met Tyr Val
        350                 355                 360

TTT TCT ACG TTT GCT AAC ATA CTG CGT AAT AAG GAG TCT         1209
Phe Ser Thr Phe Ala Asn Ile Leu Arg Asn Lys Glu Ser
        365                 370                 375

TAATAAGCTT                                                   1219
```

(2) ANGABEN ZU SEQ ID-NO:11:

(i) SEQUENZCHARAKTERISTIKA:

(A)LÄNGE: 1380 Basenpaare (B)ART: Nucleinsäure (C) STRANGFORM: Doppelstrang (D) TOPOLOGIE: linear

(ii) ART DES MOLEKÜLS: Fragment des Plasmids pBBP21

(ix) MERKMAL:

NAME/SCHLÜSSEL: Signalpeptid
LAGE: (22..84)

(ix) MERKMAL:

NAME/SCHLÜSSEL: reifes Peptid
LAGE: (85..636)
SONSTIGE ANGABEN:
/Produkt="Fusionsprotein aus Bilin-Bindungsprotein und Strep-Tag II"

(ix) MERKMAL:

NAME/SCHLÜSSEL: Signalpeptid
LAGE: (658..717)

(ix) MERKMAL:

NAME/SCHLÜSSEL: reifes Peptid
LAGE: (718..1365)
SONSTIGE ANGABEN:
/Produkt="DsbC-Protein"

(xi) SEQUENZBESCHREIBUNG: SEQ ID-NO:11:

```
TCTAGATAAC GAGGGCAAAA A ATG AAA AAG ACA GCT ATC GCG ATT 45
                         Met Lys Lys Thr Ala Ile Ala Ile
                         -21 -20                 -15


GCA GTG GCA CTG GCT GGT TTC GCT ACC GTA GCG CAG GCC GAC GTG 90
Ala Val Ala Leu Ala Gly Phe Ala Thr Val Ala Gln Ala Asp Val
            -10             -5                  -1   1


TAC CAC GAC GGT GCC TGT CCC GAA GTC AAG CCA GTC GAC AAC TTC 135
Tyr His Asp Gly Ala Cys Pro Glu Val Lys Pro Val Asp Asn Phe
            5               10                  15


GAC TGG TCC CAG TAC CAT GGT AAA TGG TGG GAA GTC GCC AAA TAC 180
Asp Trp Ser Gln Tyr His Gly Lys Trp Trp Glu Val Ala Lys Tyr
            20              25                  30


CCC AAC TCA GTT GAG AAG TAC GGA AAG TGC GGA TGG GCT GAG TAC 225
Pro Asn Ser Val Glu Lys Tyr Gly Lys Cys Gly Trp Ala Glu Tyr
            35              40                  45


ACT CCT GAA GGC AAG AGT GTC AAA GTT TCG AAC TAC CAC GTA ATC 270
Thr Pro Glu Gly Lys Ser Val Lys Val Ser Asn Tyr His Val Ile
            50              55                  60


CAC GGC AAG GAA TAC TTT ATT GAA GGA ACT GCC TAC CCA GTT GGT 315
His Gly Lys Glu Tyr Phe Ile Glu Gly Thr Ala Tyr Pro Val Gly
            65              70                  75


GAC TCC AAG ATT GGA AAG ATC TAC CAC AGC CTG ACT TAC GGA GGT 360
Asp Ser Lys Ile Gly Lys Ile Tyr His Ser Leu Thr Tyr Gly Gly
            80              85                  90


GTC ACC AAG GAG AAC GTA TTC AAC GTA CTC TCC ACT GAC AAC AAG 405
Val Thr Lys Glu Asn Val Phe Asn Val Leu Ser Thr Asp Asn Lys
            95              100                 105


AAC TAC ATC ATC GGA TAC TAC TGC AAA TAC GAC GAG GAC AAG AAG 450
Asn Tyr Ile Ile Gly Tyr Tyr Cys Lys Tyr Asp Glu Asp Lys Lys
            110             115                 120


GGA CAC CAA GAC TTC GTC TGG GTG CTC TCC AGA AGC ATG GTC CTT 495
Gly His Gln Asp Phe Val Trp Val Leu Ser Arg Ser Met Val Leu
            125             130                 135


ACT GGT GAA GCC AAG ACC GCT GTC GAG AAC TAC CTT ATC GGC TCC 540
Thr Gly Glu Ala Lys Thr Ala Val Glu Asn Tyr Leu Ile Gly Ser
            140             145                 150
```

```
CCA GTA GTC GAC TCC CAG AAA CTG GTA TAC AGT GAC TTC TCT GAA 585
Pro Val Val Asp Ser Gln Lys Leu Val Tyr Ser Asp Phe Ser Glu
        155                 160                 165

GCC GCC TGC AAG GTC AAC AAT AGC AAC TGG TCT CAC CCG CAG TTC 630
Ala Ala Cys Lys Val Asn Asn Ser Asn Trp Ser His Pro Gln Phe
        170                 175                 180

GAA AAA TAATAAGCTT CGGGAAGATT T ATG AAG AAA GGT TTT ATG 675
Glu Lys                           Met Lys Lys Gly Phe Met
                                  -20                 -15

TTG TTT ACT TTG TTA GCG GCG TTT TCA GGC TTT GCT CAG GCT GAT 720
Leu Phe Thr Leu Leu Ala Ala Phe Ser Gly Phe Ala Gln Ala Asp
            -10                 -5                  -1  1

GAC GCG GCA ATT CAA CAA ACG TTA GCC AAA ATG GGC ATC AAA AGC 765
Asp Ala Ala Ile Gln Gln Thr Leu Ala Lys Met Gly Ile Lys Ser
            5                   10                  15

AGC GAT ATT CAG CCC GCG CCT GTA GCT GGC ATG AAG ACA GTT CTG 810
Ser Asp Ile Gln Pro Ala Pro Val Ala Gly Met Lys Thr Val Leu
            20                  25                  30

ACT AAC AGC GGC GTG TTG TAC ATC ACC GAT GAT GGT AAA CAT ATC 855
Thr Asn Ser Gly Val Leu Tyr Ile Thr Asp Asp Gly Lys His Ile
            35                  40                  45

ATT CAG GGG CCA ATG TAT GAC GTT AGT GGC ACG GCT CCG GTC AAT 900
Ile Gln Gly Pro Met Tyr Asp Val Ser Gly Thr Ala Pro Val Asn
            50                  55                  60

GTC ACC AAT AAG ATG CTG TTA AAG CAG TTG AAT GCG CTT GAA AAA 945
Val Thr Asn Lys Met Leu Leu Lys Gln Leu Asn Ala Leu Glu Lys
            65                  70                  75

GAG ATG ATC GTT TAT AAA GCG CCG CAG GAA AAA CAC GTC ATC ACC 990
Glu Met Ile Val Tyr Lys Ala Pro Gln Glu Lys His Val Ile Thr
            80                  85                  90

GTG TTT ACT GAT ATT ACC TGT GGT TAC TGC CAC AAA CTG CAT GAG 1035
Val Phe Thr Asp Ile Thr Cys Gly Tyr Cys His Lys Leu His Glu
            95                  100                 105

CAA ATG GCA GAC TAC AAC GCG CTG GGG ATC ACC GTG CGT TAT CTT 1080
Gln Met Ala Asp Tyr Asn Ala Leu Gly Ile Thr Val Arg Tyr Leu
            110                 115                 120

GCT TTC CCG CGC CAG GGG CTG GAC AGC GAT GCA GAG AAA GAA ATG 1125
Ala Phe Pro Arg Gln Gly Leu Asp Ser Asp Ala Glu Lys Glu Met
            125                 130                 135

AAA GCT ATC TGG TGT GCG AAA GAT AAA AAC AAA GCG TTT GAT GAT 1170
Lys Ala Ile Trp Cys Ala Lys Asp Lys Asn Lys Ala Phe Asp Asp
            140                 145                 150
```

29

```
GTG ATG GCA GGT AAA AGC GTC GCA CCA GCC AGT TGC GAC GTG GAT 1215
Val Met Ala Gly Lys Ser Val Ala Pro Ala Ser Cys Asp Val Asp
            155                 160                 165

ATT GCC GAC CAT TAC GCA CTT GGC GTC CAG CTT GGC GTT AGC GGT 1260
Ile Ala Asp His Tyr Ala Leu Gly Val Gln Leu Gly Val Ser Gly
            170                 175                 180

ACT CCG GCA GTT GTG CTG AGC AAT GGC ACA CTT GTT CCG GGT TAC 1305
Thr Pro Ala Val Val Leu Ser Asn Gly Thr Leu Val Pro Gly Tyr
            185                 190                 195

CAG CCG CCG AAA GAG ATG AAA GAA TTC CTC GAC GAA CAC CAA AAA 1350
Gln Pro Pro Lys Glu Met Lys Glu Phe Leu Asp Glu His Gln Lys
            200                 205                 210

ATG ACC AGC GGT AAA TAATTCGCGT AGCTT 1380
Met Thr Ser Gly Lys
            215
```

(2) ANGABEN ZU SEQ ID-NO:12:

(i) SEQUENZCHARAKTERISTIKA:

(A) LÄNGE: 793 Basenpaare
(B) ART: Nucleinsäure
(C) STRANGFORM: Doppelstrang
(D) TOPOLOGIE: linear

(ii) ART DES MOLEKÜLS: Fragment des Plasmids pBBP22

(ix) MERKMAL:

NAME/SCHLÜSSEL: Signalpeptid
LAGE: (22..84)

(ix) MERKMAL:

NAME/SCHLÜSSEL: reifes Peptid
LAGE: (85..783)
SONSTIGE ANGABEN:
/Produkt="Fusionsprotein aus Bilin-Bindungsprotein, Strep-Tag II und Albumin-Bindungsdomäne"

(ix) MERKMAL:

NAME/SCHLÜSSEL: kodierende Sequenz
LAGE: (85..606)
SONSTIGE ANGABEN:
/Produkt="matures Bilin-Bindungsprotein"

(ix) MERKMAL:

NAME/SCHLÜSSEL: kodierende Sequenz
LAGE: (607..636)
SONSTIGE ANGABEN:
/Produkt="Strep-Tag II-Affinitätsanhängsel"

(ix) MERKMAL:

NAME/SCHLÜSSEL: kodierende Sequenz
LAGE: (637..783).
SONSTIGE ANGABEN:
/Produkt="Albumin-Bindungsdomäne aus Protein G"

(xi) SEQUENZBESCHREIBUNG: SEQ ID-NO:12:

```
TCTAGATAAC GAGGGCAAAA A ATG AAA AAG ACA GCT ATC GCG ATT 45
                         Met Lys Lys Thr Ala Ile Ala Ile
                         -21 -20                 -15

GCA GTG GCA CTG GCT GGT TTC GCT ACC GTA GCG CAG GCC GAC GTG 90
Ala Val Ala Leu Ala Gly Phe Ala Thr Val Ala Gln Ala Asp Val
            -10              -5              -1   1

TAC CAC GAC GGT GCC TGT CCC GAA GTC AAG CCA GTC GAC AAC TTC 135
Tyr His Asp Gly Ala Cys Pro Glu Val Lys Pro Val Asp Asn Phe
            5               10              15

GAC TGG TCC CAG TAC CAT GGT AAA TGG TGG GAA GTC GCC AAA TAC 180
Asp Trp Ser Gln Tyr His Gly Lys Trp Trp Glu Val Ala Lys Tyr
            20              25              30

CCC AAC TCA GTT GAG AAG TAC GGA AAG TGC GGA TGG GCT GAG TAC 225
Pro Asn Ser Val Glu Lys Tyr Gly Lys Cys Gly Trp Ala Glu Tyr
            35              40              45

ACT CCT GAA GGC AAG AGT GTC AAA GTT TCG AAC TAC CAC GTA ATC 270
Thr Pro Glu Gly Lys Ser Val Lys Val Ser Asn Tyr His Val Ile
            50              55              60

CAC GGC AAG GAA TAC TTT ATT GAA GGA ACT GCC TAC CCA GTT GGT 315
His Gly Lys Glu Tyr Phe Ile Glu Gly Thr Ala Tyr Pro Val Gly
            65              70              75

GAC TCC AAG ATT GGA AAG ATC TAC CAC AGC CTG ACT TAC GGA GGT 360
Asp Ser Lys Ile Gly Lys Ile Tyr His Ser Leu Thr Tyr Gly Gly
            80              85              90

GTC ACC AAG GAG AAC GTA TTC AAC GTA CTC TCC ACT GAC AAC AAG 405
Val Thr Lys Glu Asn Val Phe Asn Val Leu Ser Thr Asp Asn Lys
            95              100             105

AAC TAC ATC ATC GGA TAC TAC TGC AAA TAC GAC GAG GAC AAG AAG 450
Asn Tyr Ile Ile Gly Tyr Tyr Cys Lys Tyr Asp Glu Asp Lys Lys
            110             115             120

GGA CAC CAA GAC TTC GTC TGG GTG CTC TCC AGA AGC ATG GTC CTT 495
Gly His Gln Asp Phe Val Trp Val Leu Ser Arg Ser Met Val Leu
            125             130             135

ACT GGT GAA GCC AAG ACC GCT GTC GAG AAC TAC CTT ATC GGC TCC 540
Thr Gly Glu Ala Lys Thr Ala Val Glu Asn Tyr Leu Ile Gly Ser
            140             145             150

CCA GTA GTC GAC TCC CAG AAA CTG GTA TAC AGT GAC TTC TCT GAA 585
Pro Val Val Asp Ser Gln Lys Leu Val Tyr Ser Asp Phe Ser Glu
            155             160             165
```

```
GCC GCC TGC AAG GTC AAC AAT AGC AAC TGG TCT CAC CCG CAG TTC 630
Ala Ala Cys Lys Val Asn Asn Ser Asn Trp Ser His Pro Gln Phe
            170             175             180

GAA AAA CCA GCT AGC CTG GCT GAA GCT AAA GTT CTG GCT AAC CGT 675
Glu Lys Pro Ala Ser Leu Ala Glu Ala Lys Val Leu Ala Asn Arg
            185             190             195

GAA CTG GAC AAA TAC GGT GTT TCC GAC TAC TAC AAA AAC CTC ATC 720
Glu Leu Asp Lys Tyr Gly Val Ser Asp Tyr Tyr Lys Asn Leu Ile
            200             205             210

AAC AAC GCT AAA ACC GTT GAA GGT GTT AAA GCT CTG ATC GAC GAA 765
Asn Asn Ala Lys Thr Val Glu Gly Val Lys Ala Leu Ile Asp Glu
            215             220             225

ATT CTC GCA GCA CTG CCG TAATAAGCTT 793
Ile Leu Ala Ala Leu Pro
            230
```

(2) ANGABEN ZU SEQ ID-NO:13:

    (i) SEQUENZCHARAKTERISTIKA:

        (A) LÄNGE: 7 Aminosäuren
        (B) ART: Aminosäure
        (C) TOPOLOGIE: linear

    (ii) ART DES MOLEKÜLS: synthetisches Peptid

    (ix) MERKMAL:

        NAME/SCHLÜSSEL: sonstige Merkmale
        LAGE: 1
        SONSTIGE ANGABEN: Xaa ist Biotinamidocaproyl

    (ix) MERKMAL:

        NAME/SCHLÜSSEL: sonstige Merkmale
        LAGE: 7
        SONSTIGE ANGABEN: C-Terminus ist Amid

    (xi). SEQUENZBESCHREIBUNG: SEQ ID-NO:13:

```
Xaa Ser Pro Thr His Tyr Val
 1                   5
```

**Patentansprüche**

1. Lipocalinmutein, basierend auf einem Polypeptid der Lipocalinfamilie, bei dem Aminosäurepositionen im Bereich von drei der vier Peptidschleifen, die an einem Ende der zylindrischen Faltblattstruktur angeordnet sind, und die denjenigen Abschnitten in der linearen Polypeptidsequenz entsprechen, die die Aminosäurepositionen 28 bis 45, 58 bis 69, 86 bis 99 und 114 bis 129 des Bilin-Bindungsproteins (Bbp) aus *Pieris brassicae* umfassen, mutiert sind, und das einen vorgegebenen neuen Liganden mit bestimmbarer Affinität bindet.

2. Lipocalinmutein nach Anspruch 1, wobei das Lipocalin ausgewählt wird aus der Gruppe bestehend aus dem Bilin-Bindungsprotein aus *Pieris brassicae,* dem Retinol-Bindungsprotein des Menschen und dem Apolipoprotein D des Menschen.

3. Lipocalinmutein nach Anspruch 1 oder 2, wobei es sich bei den mutierten Aminosäurepositionen im Bereich der vier Peptidschleifen um die Sequenzpositionen 34 bis 37, 58, 60, 69, 88, 90, 93, 95, 97, 114, 116, 125 und 127 des Bilin-Bindungsproteins oder um die Sequenzpositionen 34 bis 37, 59, 61, 70, 87, 89, 92, 94, 96, 113, 115, 123 und 125 des Apolipoprotein D handelt.

4. Lipocalinmutein nach einem der Ansprüche 1 bis 3, wobei weitere Aminosäuren in dem Lipocalinmutein ausgetauscht sind, um die monomere Struktur des Lipocalinmuteins zu stabilisieren, um Erkennungsstellen für Proteasen in dem Lipocalinmutein zu eliminieren oder um geeignete Restriktionsschnittstellen zur Manipulation der für das Lipocalinmutein kodierenden Nukleinsäure einzuführen.

5. Lipocalinmutein nach einem der Ansprüche 1 bis 4, wobei der Ligand eine chemische Verbindung in freier oder konjugierter Form ist, die Merkmale eines immunologischen Haptens aufweist.

6. Lipocalinmutein nach einem der Ansprüche 1 bis 4, wobei der Ligand ein Peptid, ein Polypeptid oder ein anderes Makromolekül oder ein entsprechendes Konjugat davon ist.

7. Fusionsprotein aus einem Lipocalinmutein gemäss einem der Ansprüche 1 bis 6 und einem Fusionspartner, der ein Polypeptid oder eine Proteindomäne ist.

8. Fusionsprotein nach Anspruch 7, wobei der Fusionspartner ein Lipocalinmutein mit anderer oder derselben Ligandenspezifität ist.

9. Verfahren zur Herstellung von Lipocalinmuteinen nach einem der Ansprüche 1 bis 6, wobei die aus der Mutagenese resultierende Nukleinsäure, die für die Bibliothek der Lipocalinmuteine kodiert, zur Selektion des oder der Lipocalinmuteine auf Bindung des vorgegebenen Liganden am 3'-Ende mit einem Gen, das für das Hüllprotein pIII eines filamentösen Bakteriophagen der M13-Familie oder für ein Fragment dieses Hüllproteins kodiert, in operabler Weise fusioniert wird.

10. Verfahren zur Herstellung von Lipocalinmuteinen nach einem der Ansprüche 1 bis 6, wobei das Lipocalinmutein, ein Fragment des Lipocalinmuteins oder ein Fusionsprotein aus dem Lipocalinmutein und einem anderen Polypeptid ausgehend von der für das Lipocalinmutein kodierenden Nukleinsäure mittels gentechnischer Methoden in einem bakteriellen oder eukaryontischen Wirtsorganismus produziert wird und aus diesem Wirtsorganismus oder dessen Kultur gewonnen wird.

11. Verwendung von Lipocalinmuteinen oder von Fusionsproteinen von Lipocalinmuteinen nach einem oder mehreren der Ansprüche 1 bis 8 zur Bindung an eine Festphase, so dass der Ligand des Lipocalinmuteins oder ein Konjugat oder Fusionsprotein dieses Liganden immobilisiert oder abgetrennt werden kann.

12. Verwendung von Lipocalinmuteinen oder von Fusionsproteinen von Lipocalinmuteinen nach einem oder mehreren der Ansprüche 1 bis 8 zur Markierung mit einem Enzym, einem Antikörper, einer radioaktiven Substanz oder einer anderen Gruppe mit einer biochemischen Aktivität oder mit definierten Bindungseigenschaften, so dass der Ligand des Lipocalinmuteins oder ein Konjugat oder Fusionsprotein dieses Liganden damit nachgewiesen oder in Kontakt gebracht werden kann.

**Claims**

1. Lipocalin mutein based on a polypeptide of the lipocalin family, wherein amino acid positions in the region of three of the four peptide loops, which are arranged at one end of the cylindrical β-pleated sheet structure and correspond to those segments in the linear polypeptide sequence that comprise the amino acid positions 28 to 45, 58 to 69, 86 to 99 and 114 to 129 of the bilin-binding protein (Bbp) of *Pieris brassicae,* are mutated, and which binds a given new ligand with detectable affinity.

2. Lipocalin mutein of claim 1, wherein the lipocalin is selected from the group consisting of the bilin-binding protein of *Pieris brassicae,* human retinol-binding protein and human apolipoprotein D.

3. Lipocalin mutein of claim 1 or 2, wherein the mutated amino acid positions in the region of the four peptide loops are sequence positions 34 to 37, 58, 60, 69, 88, 90, 93, 95, 97, 114, 116, 125 and 127 of the bilin-binding protein or sequence positions 34 to 37, 59, 61, 70, 87, 89, 92, 94, 96, 113, 115, 123 and 125 of apolipoprotein D.

4. Lipocalin mutein according to any one of claims 1 to 3, wherein in the lipocalin mutein further amino acids are substituted in order to stabilize the monomeric structure of the lipocalin mutein, eliminate recognition sites for proteases within the lipocalin mutein, or introduce suitable restriction sites for the manipulation of the nucleic acid encoding the lipocalin mutein.

5. Lipocalin mutein according to any one of claims 1 to 4, wherein the ligand is a chemical compound in free or conjugated form which exhibits the features of an immunological hapten.

6. Lipocalin mutein according to any one of claims 1 to 4, wherein the ligand is a peptide, a polypeptide or another macromolecule or a corresponding conjugate thereof.

7. Fusion protein of a lipocalin mutein according to any one of claims 1 to 6 and a fusion partner, wherein the fusion partner is a polypeptide or a protein domain.

8. Fusion protein according to claim 7, wherein the fusion partner is a lipocalin mutein with different or the same ligand specificity.

9. Method for the production of lipocalin muteins according to any one of claims 1 to 6, wherein the nucleic acid resulting from the mutagenesis which encodes the library of lipocalin muteins is operably fused at the 3' end with a gene encoding the coating protein pIII of a filamentous bacteriophage of the M13-family or a fragment of this coating protein for the selection of the lipocalin mutein(s) for binding of the given ligand.

10. Method for the production of lipocalin muteins according to any one of claims 1 to 6, wherein the lipocalin mutein, a fragment of the lipocalin mutein or a fusion protein of the lipocalin mutein and another polypeptide is produced starting from the nucleic acid encoding the lipocalin mutein by means of genetic engineering methods in a bacterial or eukaryotic host organism and is isolated from this host organism or its culture.

11. Use of lipocalin muteins or fusion proteins of lipocalin muteins according to any one of claims 1 to 8 for binding to a solid phase so that the ligand of the lipocalin mutein or a conjugate or fusion protein of this ligand can be immobilized or isolated.

12. Use of lipocalin muteins or fusion proteins of lipocalin muteins according to any one of claims 1 to 8 for labeling with an enzyme, an antibody, a radioactive substance or another group with biochemical activity or with defined binding properties so that the ligand of the lipocalin mutein or a conjugate or fusion protein of this ligand can be detected or can be contacted therewith.

**Revendications**

1. Mutéine de lipocaline, basée sur un polypeptide de la famille des lipocalines, dans lequel on a muté des positions d'acides aminés situées dans la région de trois des quatre boucles peptidiques, qui sont aménagées à une extrémité de la structure de feuillets en forme de cylindre, et qui correspondent aux segments de la séquence polypeptidique linéaire qui comprennent les positions d'acides aminés 28 à 45, 58 à 69, 86 à 99 et 114 à 129 de la protéine liant la biline (Bbp) issue de l'espèce *Pieris brassicae,* et lequel lie un nouveau ligand prédéfini avec une affinité pouvant être déterminée.

2. Mutéine de lipocaline selon la revendication 1, dans laquelle la lipocaline est choisie dans le groupe constitué de la protéine liant la biline issue de l'espèce *Pieris brassicae,* de la protéine de liaison au rétinol humaine et de l'apolipoprotéine D humaine.

**3.** Mutéine de lipocaline selon la revendication 1 ou 2, dans laquelle les positions d'acides aminés mutées dans la région des quatre boucles peptidiques sont les positions correspondant aux séquences 34 à 37, 58, 60, 69, 88, 90, 93, 95, 97, 114, 116, 125 et 127 de la protéine liant la biline ou les positions correspondant aux séquences 34 à 37, 59, 61, 70, 87, 89, 92, 94, 96, 113, 115, 123 et 125 de l'apolipoprotéine D.

**4.** Mutéine de lipocaline selon l'une quelconque des revendications 1 à 3, dans laquelle d'autres acides aminés sont échangés dans la mutéine de lipocaline pour stabiliser la structure monomère de la mutéine de lipocaline, pour éliminer des sites de reconnaissance par des protéases dans la mutéine de lipocaline ou pour insérer des sites de coupure de restriction appropriés en vue de la manipulation de l'acide nucléique codant pour la mutéine de lipocaline.

**5.** Mutéine de lipocaline selon l'une quelconque des revendications 1 à 4, dans laquelle le ligand est un composé chimique sous forme libre ou conjuguée, qui présente les caractéristiques d'un haptène immunologique.

**6.** Mutéine de lipocaline selon l'une quelconque des revendications 1 à 4, dans laquelle le ligand est un peptide, un polypeptide ou une autre macromolécule ou un conjugué correspondant de ceux-ci.

**7.** Protéine de fusion issue d'une mutéine de lipocaline selon l'une quelconque des revendications 1 à 6 et d'un partenaire de fusion, qui est un polypeptide ou un domaine protéique.

**8.** Protéine de fusion selon la revendication 7, dans laquelle le partenaire de fusion est une mutéine de lipocaline ayant une spécificité à un ligand différente ou identique.

**9.** Procédé de fabrication de mutéines de lipocaline selon l'une quelconque des revendications 1 à 6, dans lequel on fusionne sous forme fonctionnelle l'acide nucléique obtenu par mutagenèse, qui code pour la bibliothèque de mutéines de lipocaline dans le but de sélectionner la ou les mutéines de lipocaline sur la liaison du ligand prédéfini à l'extrémité 3', avec un gène qui code pour la protéine d'enveloppe pIII d'un bactériophage filamenteux de la famille M13 ou pour un fragment de cette protéine d'enveloppe.

**10.** Procédé de fabrication de mutéines de lipocaline selon l'une quelconque des revendications 1 à 6, dans lequel on produit la mutéine de lipocaline, un fragment de la mutéine de lipocaline ou une protéine de fusion constituée de la mutéine de lipocaline et d'un autre polypeptide, à partir de l'acide nucléique codant pour la mutéine de lipocaline au moyen de procédés de technique génétique dans un organisme hôte bactérien ou eucaryote, et on récupère ce produit à partir de cet organisme hôte ou de sa culture.

**11.** Utilisation de mutéines de lipocaline, ou de protéines de fusion de mutéines de lipocaline, selon l'une quelconque ou plusieurs des revendications 1 à 8, pour la liaison à une phase solide, de sorte que le ligand de la mutéine de lipocaline ou un conjugué ou une protéine de fusion de ce ligand puisse être immobilisé ou séparé.

**12.** Utilisation de mutéines de lipocaline ou de protéines de fusion de mutéines de lipocaline selon l'une quelconque ou plusieurs des revendications 1 à 8, pour le marquage avec une enzyme, un anticorps, une substance radioactive ou un autre groupement ayant une activité biochimique ou ayant des propriétés de liaison définies, de sorte que le ligand de la mutéine de lipocaline, ou un conjugué ou une protéine de fusion de ce ligand, puisse être détecté ou être mis en contact avec celles-ci.

Fig. 1

Fig. 2

```
Rbp     1   ERDCRVSSFRVKENFDKARFSGTWYAMAKKDPEGLFLQDNIVAEFSV.  47
ApoD    1   QAFHLGKCPNPPVQENFDVNKYLGRWYEIEKIPTTFEN.GRCIQANYSLM  49
Bbp     1   NVYHDGACPEVKPVDNFDWSNYHGKWWEVAKYPNSVEKYGKCGWAEYTP.  49
                                                 * * * *
                                      ==================
```

```
Rbp    48   DETGQMSATAKGRVRLLNNWDVCADMVGTFTDTEDP..AKFKMKYWGVAS  95
ApoD   50   ENGKIKVLNQELRADG.....TVNQIEGEATPVNLT..EPAKLEVKFSWF  92
Bbp    50   E.GKSVKVSNYHVIHG.....KEYFIEGTAYPVGDSKIGKIYHKLTYGGV  93
                    *  *                 *                 *  *   *
                    ==================               =========
```

```
Rbp    96   FLQKGNDDHWIVDTDYDTYAVQYSCRLLNLDGTCADSYSFVFSRDPNGLP  145
ApoD   93   ...MPSAPYWILATDYENYALVYSCTCI.IQ.LFHVDFAWILARNPNL.P  136
Bbp    94   ...TKENVFNVLSTDNKNYIIGYYCKYD.EDKKGHQDFVWVLSRSKVL.T  138
                      *  *                  *  *          *  *
                    =========         ==================
```

```
Rbp   146   PEAQKIVRQRQEEL.CLA.RQYRLIVH...NGYCDGRSERNLL  183
ApoD  137   PETVDSLKNILT.SNNIDVKKMTVTD..QVNCPKLS  169
Bbp   139   GEAKTAVENYLIGSPVVDSQKLVYSDFSEAACKVNN  174
```

Fig. 3

## 1. PCR (A + B)

## 2. PCR

Fig. 4

(XbaI)

BstXI

BstXI

tet$^{p/o}$

ompA

bbp-streptagII

pIII

tlpp

HindIII

ori

pBBP20

4306 bp

f1-IG

tetR

bla

Fig. 5

Fig. 6

Fig. 7

EP 1 270 725 B1

Fig. 8

44

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4417598 A1
- DE 19641876

**In der Beschreibung aufgeführte Nicht-Patentliteratur**

- **Pervaiz ; Brew.** *FASEB J.,* 1987, vol. 1, 209-214
- **Flower.** *Biochem. J.,* 1996, vol. 318, 1-14
- **Sawyer et al.** *Nature,* 1987, vol. 327, 659
- **Newcomer et al.** *EMBO J.,* 1984, vol. 3, 1451-1454
- **Huber et al.** *J. Mol. Biol.,* 1987, vol. 195, 423-434
- **Newcomer.** *Structure,* 1993, vol. 1, 7-18
- **Bishop ; Weiner.** *Trends Biochem. Sci.,* 1996, vol. 21, 127
- **Papiz et al.** *Nature,* 1986, vol. 324, 383-385
- **Cowan et al.** *Proteins: Struct., Funct., Genet.,* 1990, vol. 8, 44-61
- **Flower.** *FEBS Lett.,* 1994, vol. 354, 7-11
- **Yang et al.** *Biochemistry,* 1994, vol. 33, 12451-12455
- **Peitsch ; Boguski.** *New Biologist,* 1990, vol. 2, 197-206
- **Hoess.** *Curr. Opin. Struct. Biol.,* 1993, vol. 3, 572-579
- **Wells ; Lowman.** *Curr. Opin. Struct. Biol.,* 1992, vol. 2, 597-604
- **Chester ; Hawkins.** *Trends Biotechnol.,* 1995, vol. 13, 294-300
- **Bird ; Walker.** *Trends Biotechnol.,* 1991, vol. 9, 132-137
- **Desplancq et al.** *Protein Eng.,* 1994, vol. 7, 1027-1033
- **Schmidt ; Skerra.** *Eur. J. Biochem.,* 1994, vol. 219, 855-863
- **Low et al.** *J. Mol. Biol.,* 1996, vol. 260, 359-368
- **Skerra et al.** *Anal. Biochem.,* 1991, vol. 196, 151-155
- **Kay et al.** Phage Display of Peptides and Proteins - A Laboratory Manual. Academic Press, 1996
- **Model ; Russel.** The Bacteriophages. Plenum Press, 1988, vol. 2, 375-456
- **Beck ; Zink.** *Gene,* 1981, vol. 16, 35-58
- **Bullock et al.** *BioTechniques,* 1987, vol. 5, 376-379
- **Schmidt et al.** *J. Mol. Biol.,* 1996, vol. 255, 753-766
- **Cogan et al.** *Eur. J. Biochem.,* 1976, vol. 65, 71-78
- **Voss.** Fluorescein Hapten: An Immunological Probe. CRC Press, 1984
- **Barbas ; Burton.** *Trends Biotechnol.,* 1996, vol. 14, 230-234
- **Kraulis.** *J. Appl. Cryst.,* 1991, vol. 24, 946-950
- **Zapun et al.** *Biochemistry,* 1995, vol. 34, 5075-5089
- **Kraulis et al.** *FEBS Lett.,* 1996, vol. 378, 190-194
- **Sambrook et al.** Molecular Cloning. A Laboratory Manual. Cold Spring Harbor Press, 1989
- **Tung ; Chow.** *Trends Genet.,* 1995, vol. 11, 128-129
- **Hengen.** *Trends Biochem. Sci.,* 1996, vol. 21, 75-76
- **Ogano et al.** *Carbohydrate Res.,* 1982, vol. 105, 69-85
- **Yanisch-Perron et al.** *Gene,* 1985, vol. 33, 103-119
- **Voss ; Skerra.** *Protein Eng.,* 1997, vol. 10, 975-982
- **Fling ; Gregerson.** *Anal. Biochem.,* 1986, vol. 155, 83-88
- **Gill ; Hippel.** *Anal. Biochem.,* 1989, vol. 182, 319-326
- **Khudyakow et al.** *Virology,* 1995, vol. 206, 666-672